# EUROPEAN PATENT SPECIFICATION

(11) **EP 4 342 995 B1**
(45) Date of publication and mention of the grant of the patent: **18.11.2026**
(21) Application number: 23207448.4
(22) Date of filing: 30.03.2007
(51) Int. Cl.: C12N 15/09, A61K 39/395, C07K 16/00, C12N 1/15, C12N 1/19, C12N 1/21, C12N 5/10, C12P 21/08

(54) **METHODS FOR CONTROLLING BLOOD PHARMACOKINETICS OF ANTIBODIES**
VERFAHREN ZUR KONTROLLE DER BLUT-PHARMAZEUTIZITÄT VON ANTIKÖRPERN
PROCÉDÉS DE RÉGULATION DE LA PHARMACOCINÉTIQUE SANGUINE D'ANTICORPS

(30) Priority: 31.03.2006 JP 2006097796
(43) Date of publication of application: 27.03.2024
(60) Divisional application: 26188565.1 / 4 793 360
(62) Document number(s) of the earlier application(s) in accordance with Art. 76 EPC: 21196629.6 / 4 001 409; 15196240.4 / 3 056 568; 07740474.7 / 2 006 381
(73) Proprietor: CHUGAI SEIYAKU KABUSHIKI KAISHA, Tokyo 115-8543 (JP)
(72) Inventor: IGAWA, Tomoyuki, Gotemba-shi, Shizuoka 412-8513 (JP); TSUNODA, Hiroyuki, Gotemba-shi, Shizuoka 412-8513 (JP); TACHIBANA, Tatsuhiko, Gotemba-shi, Shizuoka 412-8513 (JP)
(74) Representative: Vossius & Partner Patentanwälte Rechtsanwälte mbB

(56) References cited:
- US-A1- 2005 244 403
- LI BOHUA ET AL: "Construction and characterization of a humanized anti-human CD3 monoclonal antibody 12F6 with effective immunoregulation functions", IMMUNOLOGY, WILEY-BLACKWELL PUBLISHING LTD, GB, vol. 116, no. 4, 1 December 2005 (2005-12-01), pages 487 - 498, XP002600947, ISSN: 0019-2805, [retrieved on 20051007], DOI: 10.1111/J.1365-2567.2005.02247.X
- CAMELLIA W ADAMS ET AL: "Humanization of a recombinant monoclonal antibody to produce a therapeutic HER dimerization inhibitor, pertuzumab", CANCER IMMUNOLOGY, IMMUNOTHERAPY, SPRINGER, BERLIN, DE, vol. 55, no. 6, 3 September 2005 (2005-09-03), pages 717 - 727, XP019333247, ISSN: 1432-0851, DOI: 10.1007/S00262-005-0058-X
- VERHOEYEN M E ET AL: "Construction of a reshaped HMFG1 antibody and comparison of its fine specificity with that of the parent mouse antibody", IMMUNOLOGY, WILEY-BLACKWELL PUBLISHING LTD, GB, vol. 78, no. 3, 1 March 1993 (1993-03-01), pages 364 - 370, XP002425226, ISSN: 0019-2805
- M. JOSEFINA COLOMA ET AL: "Position Effects of Variable Region Carbohydrate on the Affinity and In Vivo Behavior of an Anti-(1->6) Dextran Antibody", THE JOURNAL OF IMMUNOLOGY, vol. 162, no. 4, 15 February 1999 (1999-02-15), US, pages 2162, XP055405539, ISSN: 0022-1767
- REIMANN KEITH A ET AL: "A humanized form of a CD4-specific monoclonal antibody exhibits decreased antigenicity and prolonged plasma half-life in rhesus monkeys while retaining its unique biological and antiviral properties", AIDS RESEARCH AND HUMAN RETROVIRUSES, MARY ANN LIEBERT, US, vol. 13, no. 11, 1 January 1997 (1997-01-01), pages 933 - 943, XP009112647, ISSN: 0889-2229
- HINTON PAUL R ET AL: "An engineered human IgG1 antibody with longer serum half-life", THE JOURNAL OF IMMUNOLOGY, THE AMERICAN ASSOCIATION OF IMMUNOLOGISTS, US, vol. 176, no. 1, 1 January 2006 (2006-01-01), pages 346 - 356, XP002482524, ISSN: 0022-1767
- GHETIE V ET AL: "INCREASING THE SERUM PERSISTENCE OF AN IGG FRAGMENT BY RANDOM MUTAGENESIS", NATURE BIOTECHNOLOGY, NATURE PUBLISHING GROUP, US, vol. 15, no. 7, 1 January 1997 (1997-01-01), pages 637 - 640, XP000876642, ISSN: 1087-0156, DOI: 10.1038/NBT0797-637
- DING GUIPENG ET AL: "A murine-human chimeric IgG antibody against vascular endothelial growth factor receptor 2 inhibits angiogenesis in vitro", vol. 66, no. 3, 24 July 2013 (2013-07-24), Dordrecht, pages 395 - 411, XP093147588, ISSN: 0920-9069, Retrieved from the Internet <URL:http://link.springer.com/article/10.1007/s10616-013-9587-x/fulltext.html> DOI: 10.1007/s10616-013-9587-x

## Description

### Technical Field

The present invention is as defined in the claims. The present disclosure generally relates to methods for modifying antibodies to control pharmacokinetics of the antibodies in blood, pharmaceutical compositions comprising as an active ingredient an antibody whose pharmacokinetics in blood is controlled, and methods for producing the same.

### Background Art

Since antibodies are highly stable in blood and have few adverse effects, they have drawn much attention as pharmaceuticals. There are a number of IgG type antibody pharmaceuticals available on the market and many such are currently under development (Non-patent Documents 1 and 2). Technologies for enhancing the effector function and such have been developed to produce second-generation antibody pharmaceuticals. For example, known are technologies for enhancing antibody-dependent cell-mediated cytotoxicity (ADCC) or complement-dependent cytotoxicity (CDC) through amino acid substitutions in the Fc domain of an IgG antibody (Non-patent Document 3). In addition to such amino acid substitutions that result in enhancement of the effector function, there are reports on other amino acid substitutions in the Fc domain, which prolong antibody half-life in blood (Non-patent Documents 4 and 5). Prolonging the antibody half-life in blood enables administration of antibody pharmaceuticals at reduced doses or at longer intervals and thereby results in providing low-cost, highly advantageous antibody pharmaceuticals. Specifically, the half-life in blood can be prolonged by modifying the Fc domain through amino acid substitutions that increase the affinity for the neonatal Fc receptor known as the IgG salvage receptor. Alternatively, the half-life in blood can be prolonged by shuffling the constant region CH1, CH2, and CH3 domains (Non-patent Document 6). However, the amino acid sequences of constant regions of an IgG antibody are conserved in humans, and therefore it is best to keep the number of artificial amino acid substitutions in the constant regions to a minimum from the viewpoint of antigenicity.

Reported techniques for substituting amino acids in IgG antibody variable regions include not only humanization (Non-patent Document 7) but also affinity maturation to enhance binding activity using amino acid substitutions in complementarity determining regions (CDRs) (Non-patent Document 8) and improvement of physicochemical stability through amino acid substitutions in frameworks (FRs) (Non-patent Document 9). Thus, unlike in constant regions, amino acid substitution in variable regions is a general technique for improving function and properties of an antibody. Since the CDR amino acid sequences of a humanized antibody are derived from a nonhuman animal, the risk of antigenicity need not be regarded as a problem. Alternatively, if the FR sequence is the same as that of a human antibody publicly disclosed in the Kabat Database (http://ftp.ebi.ac.uk/ pub/databases/kabat!) or the IMGT Database (http://imgt.cines.fr/), the antigenicity risk is thought to be low. However, only the above-described amino acid substitutions in the constant region Fc are so far available as methods for improving the half-life of IgG antibodies in blood. There is no report on a method for improving the half-life of IgG antibody in blood using amino acid substitution in a variable region where the risk of antigenicity is lower. The reason is that it was considered that half-life of IgG in blood strongly depends on its binding to the neonatal Fc receptor, the salvage receptor, and antigen-dependent IgG elimination (Non-patent Document 10), and variable regions have no significant influence on this half-life in blood. Meanwhile, the isoelectric point (pi) of IgG is decreased when IgG is anionized through succination (Non-patent Document 11), or the pl of IgG is increased when the antibody is cationized through modification using a polyamine (Non-patent Document 12). However, in both cases, the half-life in blood is shortened rather than prolonged. Thus, improvement of the half-life in blood has not been achieved by changing the pI through modification.

Meanwhile, the half-life of minibodies (low molecular weight antibodies) such as Fab and scFv is shorter than that of IgG, which is a whole antibody. Therefore, the half-life of minibodies in blood can be prolonged by modification using a polymer such as polyethylene glycol to reduce its renal excretion (Non-patent Document 13). In addition to the modification with a polymer, a shift of the isoelectric point (pi) has also been reported to modify the pharmacokinetics of minibodies in blood. For example, Non-patent Document 14 described that modification of anti-Tac Fab with an organic acid decreased its pi, resulting in improvement of AUC (Area Under Curve). In contrast, Non-patent Documents 15 and 16 described that modification of anti-Tac dsFv with an organic acid decreased its pi, resulting in reduction of AUC. Non-patent Document 17 demonstrated that the half-life (tl/2) and AUC of anti-Tac-scFv toxin were reduced when its pI was decreased by modifying its variable regions through amino acid substitutions. Non-patent Document 18 describes that there was almost no change in the AUC of an scFv when its pI was decreased by adding amino acids to the C terminus. Thus, the AUC of a minibody may be increased or decreased when its pI is decreased by modification or amino acid substitution. Accordingly, the half-life of minibodies in blood cannot be exactly controlled as intended by shifting the pI.
[Non-patent Document 1] Monoclonal antibody successes in the clinic, Janice M Reichert, Clark J Rosensweig, Laura B Faden & Matthew C Dewitz, Nature Biotechnology 2005 ;23: 1073 - 1078
[Non-patent Document 2] Paviou AK, Belsey MJ., The therapeutic antibodies marketto 2008., Eur J Pharm Biopharm. 2005 Apr;59(3):389-96.
[Non-patent Document 3] Kim SJ, Park Y, Hong HJ., Antibody engineering for the development of therapeutic antibodies., Mol Cells. 2005 Aug 31;20(1):17-29. Review.
[Non-patent Document 4] Hinton PR, Xiong JM, Johlfs MG, Tang MT, Keller S, Tsurushita N., An engineered human IgG1 antibody with longer serum half-life., J Immunol. 2006 Jan 1;176(1):346-56.
[Non-patent Document 5] Ghetie V, Popov S, Borvak J, Radu C, Matesoi D, Medesan C, Ober RJ, Ward ES., Increasing the serum persistence of an IgG fragment by random mutagenesis., Nat Biotechnol. 1997 Jul;15(7):637-40.
[Non-patent Document 6] Zuckier LS, Chang CJ, Scharff MD, Morrison SL., Chimeric human-mouse IgG antibodies with shuffled constant region exons demonstrate that multiple domains contribute to in vivo half-life., Cancer Res. 1998 Sep 1;58(17):3905-8.
[Non-patent Document 7] Tsurushita N, Hinton PR, Kumar S., Design of humanized antibodies: from anti-Tac to Zenapax., Methods. 2005 May;36(1):69-83.
[Non-patent Document 8] Rajpal A, Beyaz N, Haber L, Cappuccilli G, Yee H, Bhatt RR, Takeuchi T, Lerner RA, Crea R., A general method for greatly improving the affinity of antibodies by using combinatorial libraries., Proc Natl Acad Sci U S A. 2005 Jun 14;102(24):8466-71.
[Non-patent Document 9] Ewert S, Honegger A, Pluckthun A., Stability improvement of antibodies for extracellular and intracellular applications: CDR grafting to stable frameworks and structure-based framework engineering., Methods. 2004 Oct;34(2):184-99. Review
[Non-patent Document 10] Lobo ED, Hansen RJ, Balthasar JP., Antibody pharmacokinetics and pharmacodynamics., J Pharm Sci. 2004 Nov;93(11):2645-68. Review.
[Non-patent Document 11] Yamasaki Y, Sumimoto K, Nishikawa M, Yamashita F, Yamaoka K, Hashida M, Takakura Y., harmacokinetic analysis of in vivo disposition of succinylated proteins targeted to liver nonparenchymal cells via scavenger receptors: importance of molecular size and negative charge density for in vivo recognition by receptors., Pharmacol Exp Ther. 2002 May;301(2):467-77.
[Non-patent Document 12] Poduslo JF, Curran GL., olyamine modification increases the permeability of proteins at the blood-nerve and blood-brain barriers., Neurochem. 1996 Apr;66(4):1599-609.
[Non-patent Document 13] Yang K, Basu A, Wang M, Chintala R, Hsieh MC, Liu S, Hua J, Zhang Z, Zhou J, Li M, Phyu H, Petti G, Mendez M, Janjua H, Peng P, Longley C, Borowski V, Mehlig M, Filpula D., Tailoring structure-function and pharmacokinetic properties of single-chain Fv proteins by site-specific PEGylation., Protein Eng. 2003 Oct;16(10):761-70.
[Non-patent Document 14] Kobayashi H, Le N, Kim IS, Kim MK, Pie JE, Drumm D, Paik DS, Waldmann TA, Paik CH, Carrasquillo JA., The pharmacokinetic characteristics of glycolated humanized anti-Tac Fabs are determined by their isoelectric points., Cancer Res. 1999 Jan 15;59(2):422-30.
[Non-patent Document 15] Kim I, Kobayashi H, Yoo TM, Kim MK, Le N, Han ES, Wang QC, Pastan I, Carrasquillo JA, Paik CH., Lowering of pl by acylation improves the renal uptake of 99mTc-labeled anti-Tac dsFv: effect of different acylating reagents., Nucl Med Biol. 2002 Nov;29(8):795-801
[Non-patent Document 16] Kim IS, Yoo TM, Kobayashi H, Kim MK, Le N, Wang QC, Pastan I, Carrasquillo JA, Paik CH., Chemical modification to reduce renal uptake of disulfide-bonded variable region fragment of anti-Tac monoclonal antibody labeled with 99mTc., Bioconjug Chem. 1999 May-Jun;10(3):447-53.
[Non-patent Document 17] Onda M, Nagata S, Tsutsumi Y, Vincent JJ, Wang Q, Kreitman RJ, Lee B, Pastan I. , Lowering the isoelectric point of the Fv portion of recombinant immunotoxins leads to decreased nonspecific animal toxicity without affecting antitumor activity., Cancer Res. 2001 Jul 1;61(13):5070-7.
[Non-patent Document 18] Pavlinkova G, Beresford G, Booth BJ, Batra SK, Colcher D., Charge-modified single chain antibody constructs of monoclonal antibody CC49: generation, characterization, pharmacokinetics, and biodistribution analysis., Nucl Med Biol. 1999 Jan;26(1):27-34.
[Non-patent Document 19] Deen WM, Lazzara MJ, Myers BD. , Structural determinants of glomerular permeability., Am J Physiol Renal Physiol. 2001 Oct;281(4):F579-96.
[Non-patent Document 20] Schaeffer RC Jr, Gratrix ML, Mucha DR, Carbajal JM., The rat glomerular filtration barrier does not show negative charge selectivity., Microcirculation. 2002 Oct;9(5):329-42.
[Non-patent Document 21] Goode NP, Shires M, Davison AM., The glomerular basement membrane charge-selectivity barrier: an oversimplified concept?, Nephrol Dial Transplant. 1996 Sep;11(9):1714-6.
[Non-patent Document 22] Comper WD, Glasgow EF., Charge selectivity in kidney ultrafiltration., Kidney Int. 1995 May;47(5):1242-51.
[Non-patent Document 23] Ghetie V, Ward ES. FcRn: the MHC class I-related receptor that is more than an IgG transporter. Immunol Today. 1997 Dec;18(12):592-8.
[Non-patent Document 24] He XY, Xu Z, Melrose J, Mullowney A, Vasquez M, Queen C, Vexler V, Klingbeil C, Co MS, Berg EL. Humanization and pharmacokinetics of a monoclonal antibody with specificity for both E- and P-selectin. J Immunol. 1998 Jan 15;160(2):1029-35.
[Non-patent Document 25] Gobburu JV, Tenhoor C, Rogge MC, Frazier DE Jr, Thomas D, Benjamin C, Hess DM, Jusko WJ. Pharmacokinetics/dynamics of 5c8, a monoclonal antibody to CD154 (CD40 ligand) suppression of an immune response in monkeys. J Pharmacol Exp Ther. 1998 Aug;286(2):925-30.
[Non-patent Document 26] Kashmiri SV, Shu L, Padlan EA, Milenic DE, Schlom J, Hand PH., Generation, characterization, and in vivo studies of humanized anticarcinoma antibody CC49., Hybridoma. 1995 Oct;14(5):461-73. [Non-patent Document 27] Graves SS, Goshorn SC, Stone DM, Axworthy DB, Reno JM, Bottino B, Searle S, Henry A, Pedersen J, Rees AR, Libby RT., Molecular modeling and preclinical evaluation of the humanized NR-LU-13 antibody., Clin Cancer Res. 1999 Apr;5(4):899-908.
[Non-patent Document 28] Couto JR, Blank EW, Peterson JA, Ceriani RL., Anti-BA46 monoclonal antibody Mc3: humanization using a novel positional consensus and in vivo and in vitro characterization., Cancer Res. 1995 Apr 15;55(8):1717-22.
[Non-patent Document 29] Adams CW, Allison DE, Flagella K, Presta L, Clarke J, Dybdal N, McKeever K, Sliwkowski MX. Humanization of a recombinant monoclonal antibody to produce a therapeutic HER dimerization inhibitor, pertuzumab., Cancer Immunol Immunother. 2005 Sep 3;:1-11
[Non-patent Document 30] Binz HK, Amstutz P, Pluckthun A., Engineering novel binding proteins from nonimmu-noglobulin domains., Nat Biotechnol. 2005 Oct;23(10):1257-68.

### Disclosure of the Invention

### [Problems to be Solved by the Invention]

The present invention was achieved in view of the above circumstances. An objective of the present invention is to provide methods for controlling the blood half-life of humanized IgG antibodies comprising an FcRn-binding domain by modifying the antibodies through substitution of amino acid residues exposed on their surface. The disclosure also relates to pharmaceutical compositions that comprise modified humanized IgG antibodies comprising an FcRn-binding domain whose half-life in blood is controlled by amino acid substitutions; and methods for producing the pharmaceutical compositions.

### [Means for Solving the Problems]

The present inventors conducted dedicated studies on methods for controlling the blood half-life of polypeptides comprising an FcRn-binding domain through amino acid substitutions. As a result, the present inventors developed methods for controlling the half-life of IgG antibodies by controlling the surface charge through modification of residues exposed on the surface in the variable regions of the IgG antibodies, polypeptides comprising an FcRn-binding domain. Specifically, the present inventors discovered modification sites in variable regions to control the surface charge and the half-life of IgG antibodies in blood without influencing antibody structure and function. Furthermore, the present inventors confirmed that antibodies whose half-life in blood is controlled by the present invention actually retain their activity.

Regardless of the type of target antigen, the methods of the present invention are widely applicable to humanized IgG antibodies comprising an FcRn-binding domain which are recycled via the FcRn salvage pathway, and whose major metabolic pathway is not renal excretion.

The present invention relates to methods for controlling the blood half-life of humanized IgG antibodies comprising an FcRn-binding domain by modifying the polypeptides through substitution of amino acid residues exposed on their surface. The disclosure also relates to pharmaceutical compositions that comprise modified humanized IgG antibodies comprising an FcRn-binding domain, whose half-life in blood is controlled by amino acid substitutions; and methods for producing the pharmaceutical compositions.

More specifically, the present invention is as defined in the claims.

### Brief Description of the Drawings

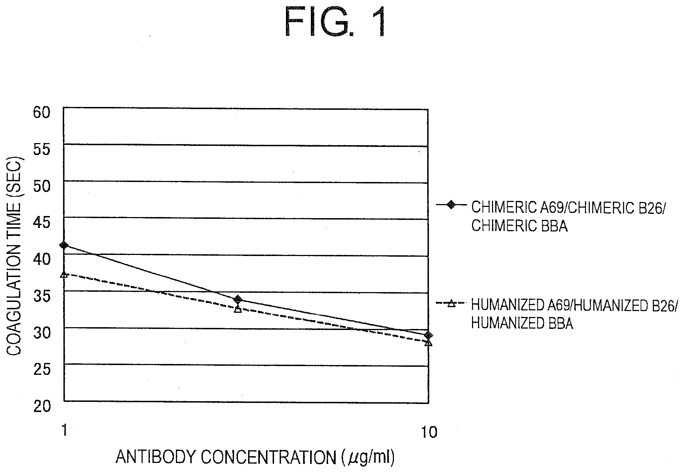

Fig. 1 is a graph showing the assessment of coagulation activity for a humanized bispecific antibody (humanized A69 (hA69a), humanized B26 (hB26-F123e4), and humanized BBA (hAL-F123j4)). The assessment result demonstrates that the coagulation activities are equivalent to or greater than those of chimeric bispecific antibodies.

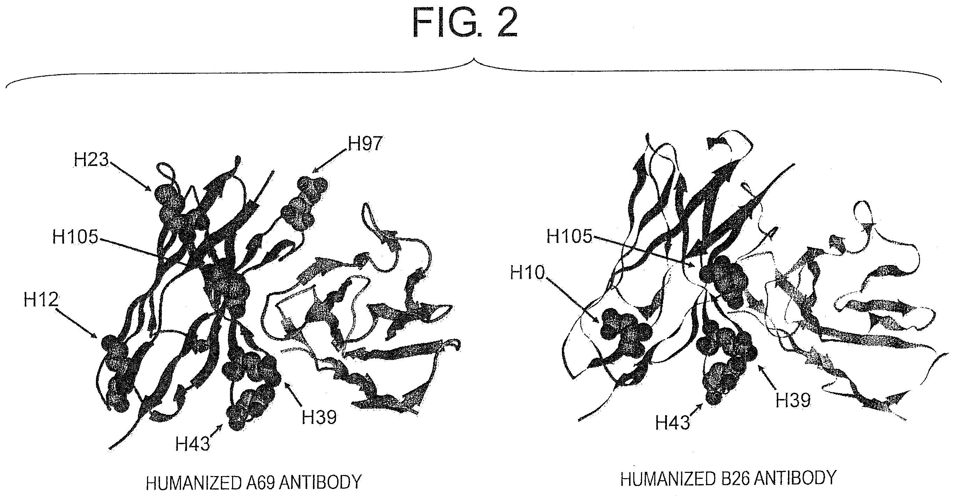

Fig. 2 is a diagram showing antibody modeling for the combinations of humanized A69-H chain variable region (hA69a) and humanized BBA (hAL-F123j4), and humanized hB26-H chain variable region (hB26-F123e4) and humanized BBA (hAL-F123j4). The side chains of amino acids that can change the surface charge are shown emphasized. The numbering was done according to the Kabat database numbering system (Kabat EA et al., (1991) Sequences of Proteins of Immunological Interest. NIH).

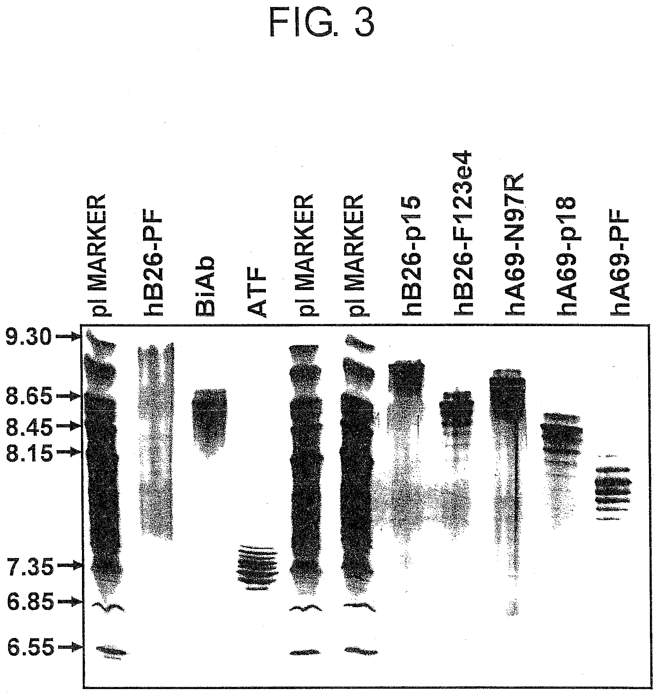

Fig. 3 is a photograph showing a result of isoelectric focusing analysis of ATF, hA69-PF, BiAb, hB26-PF, hA69-N97R, hA69-p18, hB26-F123e4, and hB26-p15.

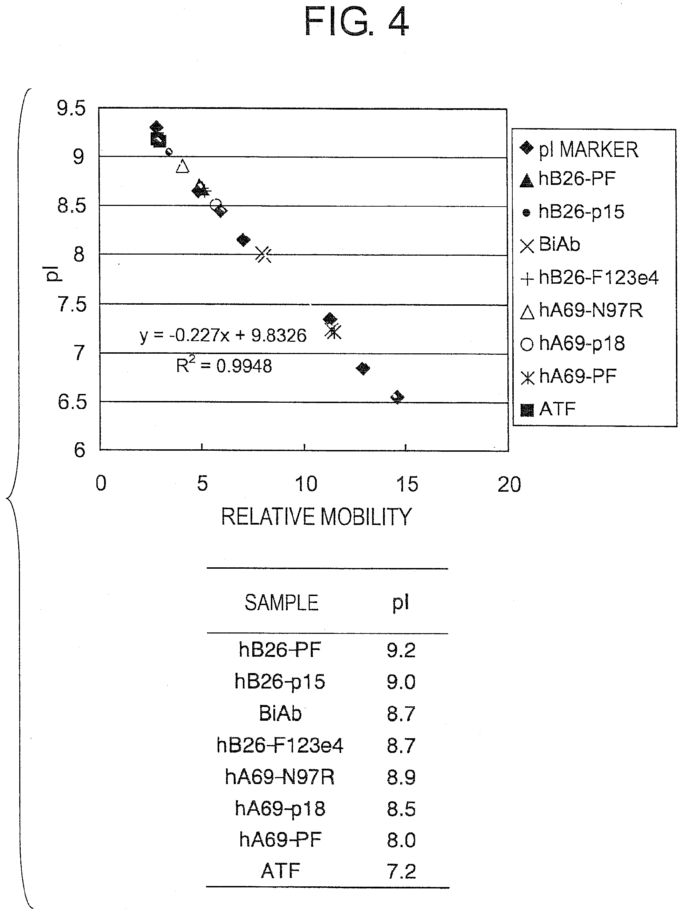

Fig. 4 shows a calibration curve of a pI marker and the pI of each sample determined from the curve, which were obtained from the isoelectric focusing analysis of ATF, hA69-PF, BiAb, hB26-PF, hA69-N97R, hA69-p18, hB26-F123e4, and hB26-p15. The diagrams demonstrate that the surface charge varies depending on the amino acid sequences of variable regions and differences in the surface charge resulting from amino acid modifications shift the pl.

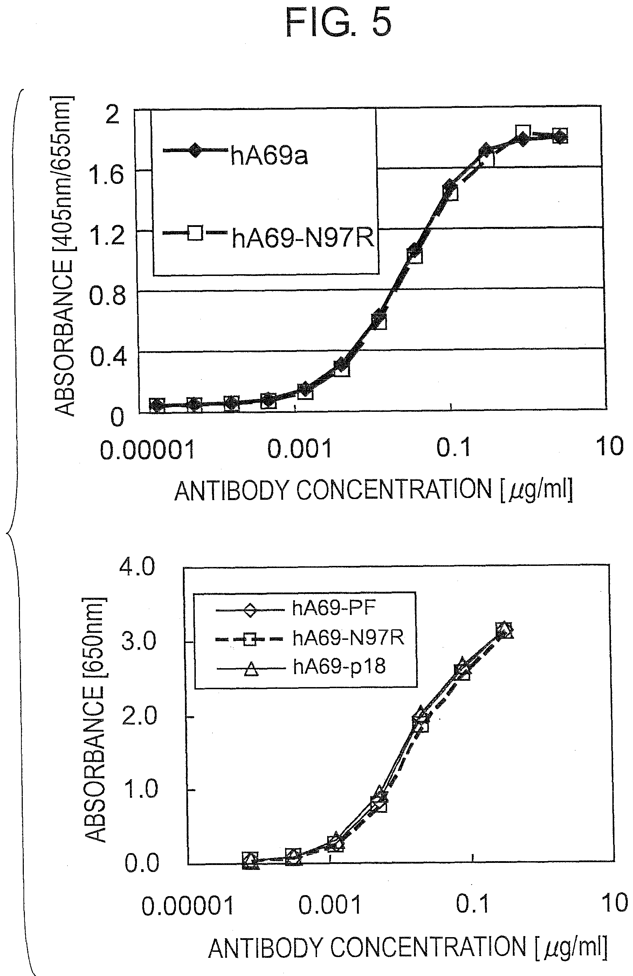

Fig. 5 shows results of analyzing humanized A69 antibodies (hA69a and hA69-N97R; hA69-N97R, hA69-p18, and hA69-PF) with unmodified or modified variable regions for their binding activity to the antigen Factor IXa. The results demonstrate that the modified antibodies with shifted isoelectric points have a binding activity comparable to that of unmodified antibodies.

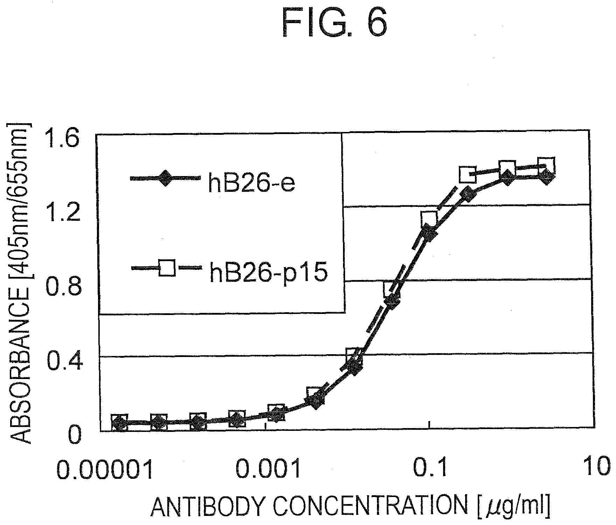

Fig. 6 is a graph showing a result of analyzing humanized B26 antibodies (hB26-F123e4 and hB26-p15) with unmodified or modified variable regions, respectively, for their binding activity to the antigen Factor X. The results demonstrate that the modified antibody with shifted isoelectric point has a binding activity comparable to that of the unmodified antibody.

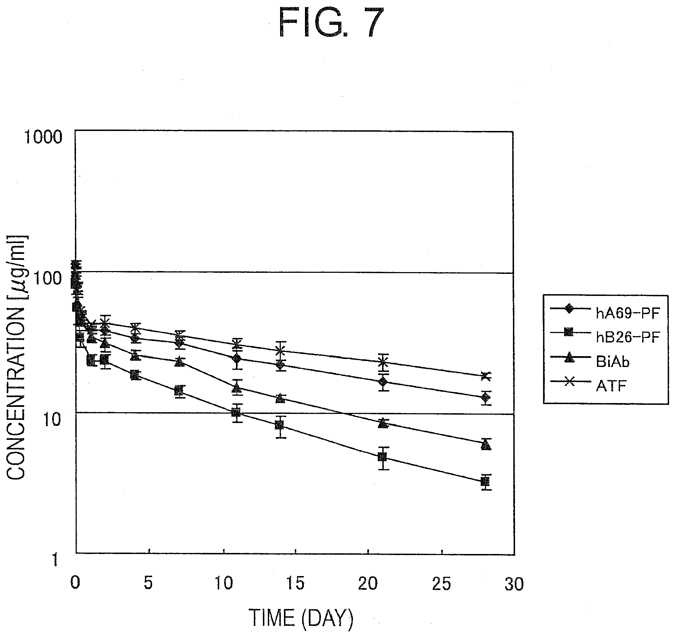

Fig. 7 is a graph showing time courses of plasma concentrations of ATF, hA69-PF, BiAb, and hB26-PF.

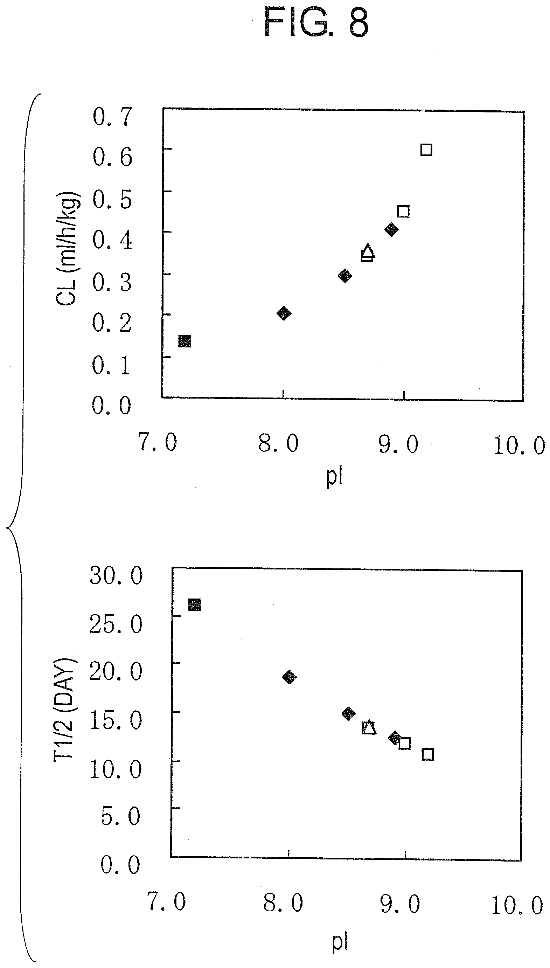

Fig. 8 shows the correlation between pl and pharmacokinetic parameters, clearance (CL) and half-life (T1/2), for ATF, hA69-PF, BiAb, hB26-PF, hA69-N97R, hA69-p18, hB26-F123e4, and hB26-p15.

### Best Mode for Carrying Out the Invention

The present invention provides methods for controlling pharmacokinetics of modified humanized IgG antibodies comprising an FcRn-binding domain in blood. In a preferred embodiment, the methods of the present invention comprise changing the charge of at least one amino acid residue that can be exposed on the surface of such polypeptides. Specifically, pharmacokinetics of these polypeptides in blood can be controlled by shifting their isoelectric point (pI) by changing the charge of their amino acid residues.

As described above, blood pharmacokinetics of minibodies such as scFvs and Fabs cannot necessarily be controlled by shifting pl. Renal excretion is known to be the major metabolic pathway of such minibodies. However, some of Non-patent Documents 19 to 22 describe that renal filtration efficiency in renal excretion is lower if protein charge is more negative, while others report that protein charge has no influence on renal filtration efficiency. In addition, some of Non-patent Documents 14 to 18 report that the half-life of a minibody in blood can be prolonged by decreasing its pI, while others report that it can be shortened by decreasing its pI. Proteins filtered through the kidney are reabsorbed by the proximal tubule. This reabsorption can become more suppressed when the charge of a protein is more negative. This suggests that the half-life of a minibody cannot be exactly controlled as intended by shifting the pl.

On the other hand, the major metabolic pathway of IgG antibodies is not renal excretion because their molecular weight is quite high. IgG antibodies with Fc are known to be recycled via the FcRn salvage pathway expressed in the endothelial cells of blood vessels and such, and thereby have a long half-life. IgG is assumed to be metabolized primarily in endothelial cells (Non-patent Document 23). Accordingly, it has been speculated that free IgG molecules are metabolized while IgG molecules non-specifically incorporated into endothelial cells are recycled via binding to FcRn. IgGs with decreased FcRn-binding activity have a shorter half-life in blood, while their half-life in blood can be prolonged by increasing their FcRn-binding activity (Non-patent Document 23). Accordingly, previous methods for controlling pharmacokinetics of IgGs in blood have been conducted by altering FcRn-binding activity through Fc modification. In contrast, Example 8 herein demonstrates that when IgGs share the same Fc domain, the IgG half-life correlates to pI showing a high correlation coefficient regardless of the type of target antigen, and that the half-life in blood of two types of antibodies against different antigens can actually be controlled by modifying the pIs of their variable regions without modifying Fc. The rate of nonspecific antibody uptake by endothelial cells is assumed to depend on the physicochemical Coulomb interaction between an IgG and a negatively charged cell surface. Therefore, it is thought that a decrease (an increase) in the pl of IgG reduces (enhances) the Coulomb interaction, and this is followed by a reduction (an increase) in nonspecific uptake by endothelial cells, leading to a decrease (an increase) in metabolism in these cells, and as a result, pharmacokinetics in blood can be controlled. Since the Coulomb interaction between endothelial cells and negatively-charged cell surface is a physicochemical interaction, the interaction is assumed not to depend on the target antigen. Thus, the methods of the present invention for controlling pharmacokinetics in blood are widely applicable to humanized IgG antibodies comprising an FcRn-binding domain which are recycled via the FcRn salvage pathway and whose major metabolic pathway is not renal excretion, regardless of the type of antigen.

Thus, the polypeptides of the present invention comprising an FcRn-binding domain are humanized IgG antibodies that can bind (has binding activity or affinity) to an Fc receptor (FcRn). Thus, the polypeptides of the present invention comprising an FcRn-binding domain are preferably proteins comprising an antibody Fc domain or Fc-like domain. The polypeptides of the present invention comprising an FcRn-binding domain are humanized IgG antibodies. Furthermore, the polypeptides of the present invention comprising an FcRn-binding domain include modified forms of antibodies (proteins) as long as they can bind to FcRn. The most preferred examples of polypeptides of the present invention comprising an FcRn-binding domain are humanized IgG antibodies.

When an IgG antibody is used as a polypeptide comprising an FcRn-binding domain in the present invention, it may be of any IgG subtype or a bispecific IgG antibody. Bispecific antibodies are antibodies specific to two different epitopes, and include antibodies that recognize different antigens and those that recognize different epitopes on a single antigen. On the other hand, when the antibody is a minibody such as scFv or Fab, whose major metabolic pathway is renal excretion, its pharmacokinetics in blood cannot be controlled by shifting pI, as described above. The present invention is applicable to any type of antibody, as long as it is an Fc-binding protein whose major metabolic pathway is not renal excretion, for example, scFv-Fc, dAb-Fc, and Fc fusion proteins. Since renal excretion is not the major metabolic pathway of these molecules, their pharmacokinetics in blood can be controlled by shifting the pI using the methods of the present invention. Antibody molecules to which the present invention is applicable may be antibody-like molecules. Antibody-like molecules refer to molecules that exert their functions by binding to target molecules (Non-patent Document 30), and include, for example, DARPins, Affibodies, and Avimers.

Herein, the phrase "pharmacokinetics in blood is controlled" indicates that pharmacokinetics in blood is shifted in a desired direction when compared between before and after modification of a polypeptide comprising an FcRn-binding domain. Specifically, when the purpose is to prolong the half-life in blood, the control of pharmacokinetics in blood means prolongation of the half-life in blood. Alternatively, when the purpose is to reduce the half-life in blood, the control of pharmacokinetics in blood refers to reduction of the half-life in blood.

In the present invention, whether the blood pharmacokinetics of a polypeptide comprising an FcRn-binding domain is shifted in a desired direction, or whether the blood pharmacokinetics can be exactly controlled as intended, can be assessed by conducting appropriate kinetic tests, for example, tests using mice, rats, rabbits, dogs, monkeys or such. More specifically, as used herein, "control of pharmacokinetics in blood" includes the control of any parameter such as half-life in blood, mean residence time in blood, or blood clearance (Pharmacokinetics: Enshu niyoru Rikai (Understanding through Exercises), Nanzando). For example, the control of pharmacokinetics in blood can be assessed by appropriate noncompartmental analysis using *in vivo* kinetics analysis software, WinNonlin (Pharsight), according to the attached instruction manual.

Herein, the phrase "amino acids that can be exposed on the surface" generally means amino acids which constitute a polypeptide comprising an FcRn-binding domain and which are present on the polypeptide surface. The side chains of an amino acid that is present on the polypeptide surface can contact solvent molecules (typically, water molecules). However, not the whole side chain has to be in contact with solvent molecules. When even a portion of the side chains contacts solvent molecules, the amino acid is judged to be present on the polypeptide surface. Those skilled in art can prepare homology models for polypeptides and antibodies through homology modeling using commercially available software, and so on, and they can also select appropriate residues as surface amino acids using the models.

In the present invention, "amino acids that can be exposed on the surface" are not particularly limited; however, they are preferably outside an FcRn-binding domain of a polypeptide comprising such a domain. An FcRn-binding domain includes, for example, Fc and Fc-like domains.

When a polypeptide of the present invention comprising an FcRn-binding domain is an IgG, amino acid residues whose charges are to be changed according to the present invention are preferably present within the heavy or light chain variable regions of the IgG antibody. Specifically, the variable regions include complementarity determining regions (CDRs) and framework regions (FRs).

Those skilled in art can select appropriate surface amino acids in antibody variable regions using homology models prepared by homology modeling or the like. Such surface amino acids include, for example, amino acids at H1, H3, H5, H8, H10, H12, H13, H15, H16, H19, H23, H25, H26, H39, H42, H43, H44, H46, H68, H71, H72, H73, H75, H76, H81, H82b, H83, H85, H86, H105, H108, H110, and H112 in an H chain FR region, but are not limited thereto in the present invention.

Likewise, those skilled in the art can also select surface amino acids in an H chain CDR region using homology models. For example, the amino acid at H97 is exposed on the surface in almost all antibodies. Surface amino acids in an L chain FR region include, for example, the amino acids at L1, L3, L7, L8, L9, L11, L12, L16, L17, L18, L20, L22, L38, L39, L41, L42, L43, L45, L46, L49, L57, L60, L63, L65, L66, L68, L69, L70, L74, L76, L77, L79, L80, L81, L85, L100, L103, L105, L106, L107, and L108, but are not limited thereto in the present invention. Likewise, those skilled in art can also select surface amino acids in an L chain CDR region using homology models.

In the methods of the present invention, "modification" of an amino acid residue specifically refers to substitution of the original amino acid residue with a different amino acid residue, deletion of the original amino acid residue, addition of another amino acid residue, and so on. The modification preferably means substitution of the original amino acid residue with a different amino acid residue. Specifically, as used herein, "modification of the charge of an amino acid residue" preferably includes amino acid substitutions.

When a polypeptide of the present invention comprising an FcRn-binding domain is an IgG antibody, the above-described "changing the charge of an amino acid residue" includes, for example, changing the charge of at least one amino acid residue selected from the group consisting of amino acid residues at positions 10, 12, 23, 39, 43, and 105 according to the Kabat's numbering system in a heavy chain of the IgG antibody. Of the amino acid residues at the positions indicated above, amino acid residues other than the amino acid residues whose charges have been modified need not be modified as long as the pharmacokinetics in blood have been controlled as intended. The amino acids can be modified to have a charge of the same type as that of modified amino acid residues or not to have any charge.

Amino acids are known to include charged amino acids. Generally known amino acids with a positive charge (positively-charged amino acids) include lysine (K), arginine (R), and histidine (H). Known amino acids with a negative charge (negatively-charged amino acids) include aspartic acid (D) and glutamic acid (E). Amino acids other than these are known as uncharged amino acids.

Preferably, the above-described "modified amino acid residues" are appropriately selected from amino acid residues of either group (a) or (b) indicated below; however, the modified amino acids are not particularly limited thereto.
(a) glutamic acid (E) and aspartic acid (D)
(b) lysine (K), arginine (R), and histidine (H)

When an original (unmodified) amino acid residue already has a charge, modification of the amino acid into an uncharged amino acid is also a preferred embodiment of the present invention. Specifically, the modification in the present invention includes:
(1) substitution of a charged amino acid with an uncharged amino acid;
(2) substitution of a charged amino acid with an amino acid carrying a charge opposite from the original amino acid; and
(3) substitution of an uncharged amino acid with a charged amino acid.

In the methods of the present invention, preferably, amino acid residues in polypeptides comprising an FcRn-binding domain are modified to shift the isoelectric point (pl). When the number of amino acid residues to be introduced through the modification is two or more, a few of them may be uncharged amino residues.

The number of amino acid residues to be modified in the methods of the present invention is not particularly limited. However, when an antibody variable region is modified for instance, only a minimum number of amino acid residues required to achieve the blood pharmacokinetics controlled as intended are preferably modified so as not to reduce the antigen-binding activity and not to increase the antigenicity.

The amino acid sequence after modification is preferably a human sequence so as not to increase the antigenicity; however, the present invention is not limited thereto. Furthermore, mutations may be introduced at sites other than those where modifications have been made for shifting the isoelectric point, so that the respective FRs (FR1, FR2, FR3, and FR4) after modification are human sequences. Such a method for substituting each FR with a human sequence has been reported in a non-patent document (Ono K et al., Mol Immunol. 1999 Apr; 36(6): 387-395). Alternatively, to shift the isoelectric point, each FR may be modified into another human FR with a different isoelectric point (for example, FR3 may be substituted by another human FR with a lower isoelectric point). Such a humanization method has been reported in a non-patent document (Dall'Acqua WF, Methods. 2005 May; 36(1): 43-60).

Even when pharmacokinetics in blood cannot be controlled as intended by modifying only a small number of surface charges, a desired polypeptide comprising an FcRn-binding domain, whose pharmacokinetics in blood is controlled, can be obtained by repeating surface charge modifications and assessment of pharmacokinetics in blood.

In rhesus monkeys, Non-patent Document 24 reports on a comparison of blood pharmacokinetics between chimeric EP5C7.g4, a chimeric antibody (IgG4), and HuEP5C7.g4, a humanized antibody (IgG4), both of which are derived from an anti-E, P-Selectin antibody, which found that the pharmacokinetics were comparable to each other. Non-patent Document 25 describes a comparison of blood pharmacokinetics in cynomolgus monkeys between ch5d8, a chimeric antibody, and Hu5c8, a humanized antibody, both of which are derived from an anti-CD 154 antibody, which found the pharmacokinetics to be comparable to each other. Non-patent Document 26 demonstrates that blood pharmacokinetics in mice of cCC49, a chimeric antibody, was comparable to that of HuCC49, a humanized antibody. Furthermore, Non-patent Documents 27 and 28 reports that pharmacokinetics and distribution in blood of mouse antibodies were comparable to those of humanized antibodies when assessed using mice. Since both mouse Fcs and human Fcs are reactive to mouse FcRn, the above findings suggest that pharmacokinetics and distribution in blood of chimeric antibodies are comparable to those of the humanized antibodies described above. As shown by these examples, pharmacokinetics of chimeric antibodies in blood are comparable to that of humanized antibodies. Specifically, when an antibody is humanized by a known method such as that described in Non-patent Document 7, its pharmacokinetics in blood is comparable to that of a chimeric antibody. Thus, humanized antibodies whose pharmacokinetics in blood is controlled cannot be produced by known methods.

Humanized antibodies whose pharmacokinetics in blood is controlled (specifically, the half-life in blood is prolonged or pharmacokinetics in blood is reduced) as compared to chimeric antibodies can be produced by shifting their pls through modification of surface amino acids at the time of humanization of chimeric antibodies, using the methods discovered by the present invention. The modification of surface amino acids to control pharmacokinetics in blood may be made at the time of humanization or after humanization.

Non-patent Document 29 describes that pharmacokinetics in blood of three types of humanized antibodies, trastuzumab, bevacizumab, and pertuzumab, obtained through humanization using the same FR sequence of a human antibody, was nearly the same. Specifically, pharmacokinetics in blood is nearly the same when humanization is performed using the same FR sequence. The blood concentration can be controlled only when the pls of antibodies are shifted by modifying surface amino acids using the methods discovered by the present invention, in addition to the above-described humanization process.

Furthermore, human antibodies whose pharmacokinetics in blood is controlled (specifically, the half-life in blood is prolonged or pharmacokinetics in blood is reduced) as compared to the original human antibodies can be produced by shifting the pls of human antibodies prepared from human antibody libraries or mice producing human antibodies and such, through modification of surface amino acids.

The "antibodies" of the present invention include antibodies obtained by further introducing amino acid substitutions, deletions, additions and/or insertions and such into the amino acid sequences of antibodies that have been already modified to change the charges of their amino acid residues as described above. The antibodies of the present invention also include antibodies obtained by further changing the charge of the amino acid residues in antibodies whose amino acid sequences have been already modified by amino acid substitutions, deletions, additions and/or insertions, chimerization, humanization or such.

Amino acid modifications such as amino acid substitutions, deletions, additions and/or insertions, and chimerization and humanization, can be achieved by methods known to those skilled in the art. Likewise, the amino acid sequences of antibody constant and variable regions that are used to produce antibodies of the present invention as recombinant antibodies may also be modified by amino acid substitutions, deletions, additions and/or insertions, or chimerization, humanization or such.

The antibodies of the present invention may be antibodies derived from any animal such as a mouse, human, rat, rabbit, goat, or camel. Furthermore, the antibodies in the context of the present disclosure may be modified antibodies, for example, chimeric antibodies. In the context of the present invention, the antibodies are particularly humanized antibodies that comprise amino acid substitutions in their sequence. The antibodies also include antibody modification products linked to various molecules.

"Chimeric antibodies" are antibodies prepared by combining sequences derived from different animals. Chimeric antibodies include, for example, antibodies comprising heavy and light chain variable (V) regions from a mouse antibody and heavy and light chain constant (C) regions from a human antibody. Chimeric antibodies can be prepared by known methods, for example, by the following procedure: a DNA encoding an antibody V region is ligated with a DNA encoding a human antibody C region; the resulting ligation product is inserted into an expression vector; and the construct can be introduced into a host to produce a chimeric antibody.

"Humanized antibodies" are also referred to as reshaped human antibodies, and can be obtained by substituting the complementarity determining regions (CDRs) of a human antibody for the CDRs of an antibody derived from a nonhuman mammal, for example, a mouse. Methods for identifying CDRs are known (Kabat et al., Sequence of Proteins of Immunological Interest (1987), National Institute of Health, Bethesda, Md.; Chothia et al., Nature (1989) 342: 877). General genetic recombination techniques for the above procedure are also known (see European Patent Application Publication No. EP 125023, and WO 96/02576). For example, the CDRs of a mouse antibody are determined by known methods, and a DNA is prepared so that it encodes an antibody in which the CDRs are linked to the framework regions (FRs) of a human antibody. A humanized antibody can then be produced by a system using a conventional expression vector. Such DNAs can be synthesized by PCR using as primers several oligonucleotides designed to comprise portions that overlap the ends of both the CDR and FR regions (see the method described in WO 98/13388). Human antibody FRs linked via CDRs are selected such that the CDRs can form a suitable antigen binding site. If required, amino acids in the FRs of an antibody variable region may be modified so that the CDRs of a reshaped human antibody can form a suitable antigen binding site (Sato, K. et al., Cancer Res. (1993) 53: 851-856). Modifiable amino acid residues in the FRs include portions that directly bind to an antigen via noncovalent bonds (Amit et al., Science (1986) 233: 747-53), portions that have an impact or effect on the CDR structure (Chothia et al., J. Mol. Biol. (1987) 196: 901-17), and portions involved in the interaction between VH and VL (EP 239400).

When the antibodies of the present invention are chimeric antibodies, which are disclosed but not claimed, or humanized antibodies, the C regions of these antibodies are preferably derived from human antibodies. For example, Cy1, Cy2, Cy3, and Cy4 can be used for the H chains, and Cκ and Cκ can be used for the L chains. Meanwhile, the human antibody C regions may be modified as required to improve antibody or production stability. A chimeric antibody, which Is disclosed but not claimed, preferably comprises a variable region of an antibody derived from a nonhuman mammal and a constant region derived from a human antibody. A humanized antibody of the present invention preferably comprises CDRs of an antibody derived from a nonhuman mammal and FRs and C regions derived from a human antibody. Constant regions of human antibodies comprise amino acid sequences specific to each antibody isotype, for example, IgG (IgG1, IgG2, IgG3, and IgG4), IgM, IgA, IgD, and IgE. The constant regions used to prepare the humanized antibodies of the present invention may be the constant regions of antibodies of any isotype. A constant region of a human IgG is preferably used, but the constant regions are not limited thereto. FRs derived from a human antibody, which are used to prepare humanized antibodies, are not particularly limited, and thus may be derived from an antibody of any isotype.

The variable and constant regions of chimeric, which are disclosed but not claimed, or humanized antibodies of the present invention may be modified by deletions, substitutions, insertions, and/or additions, as long as the antibodies exhibit the same binding specificity as that of the original antibodies.

Chimeric and humanized antibodies using human-derived sequences are expected to be useful when administered to humans for therapeutic purposes or such, since their antigenicity in the human body has been reduced.

Known sequences can be used as genes encoding the H chain or L chain of antibodies before introduction of mutations by methods of the present invention (herein, they may be simply referred to as "antibodies of the present invention"), or the genes can be obtained by methods known to those skilled in the art. For example, they may be obtained from an antibody library, or by cloning genes encoding the antibodies from hybridomas producing monoclonal antibodies.

Regarding antibody libraries, many antibody libraries are already well known, and since methods for producing antibody libraries are known, those skilled in the art can appropriately obtain antibody libraries. For example, with regard to antibody phage libraries, one can refer to the literature such as Clackson et al., Nature 1991, 352: 624-8; Marks et al., J. Mol. Biol. 1991, 222: 581-97; Waterhouses et al., Nucleic Acids Res. 1993, 21: 2265-6; Griffiths et al., EMBO J. 1994, 13: 3245-60; Vaughan et al., Nature Biotechnology 1996, 14: 309-14; and Japanese Patent Kohyo Publication No. H20-504970 (unexamined Japanese national phase publication corresponding to a non-Japanese international publication). In addition, known methods such as methods that use eukaryotic cells as libraries (WO95/15393) and ribosome display methods, may be used. Furthermore, techniques to obtain human antibodies by panning using human antibody libraries are also known. For example, variable regions of human antibodies can be expressed on the surface of phages as single chain antibodies (scFvs) using phage display methods, and phages that bind to antigens can be selected. Genetic analysis of the selected phages can determine the DNA sequences encoding the variable regions of human antibodies that bind to the antigens. Once the DNA sequences of scFvs that bind to the antigens are revealed, suitable expression vectors can be produced based on these sequences to obtain human antibodies. These methods are already well known, and one can refer to WO92/01047, WO92/20791, WO93/06213, WO93/11236, WO93/19172, WO95/01438, and WO95/15388.

As for methods for obtaining genes encoding antibodies from hybridomas, known techniques may be used, involving the use of desired antigens or cells expressing the desired antigens as sensitizing antigens, using these to perform immunizations according to conventional immunization methods, fusing the immune cells thus obtained with known parent cells by ordinary cell fusion methods, screening monoclonal antibody producing cells (hybridomas) by ordinary screening methods, synthesizing cDNAs of antibody variable regions (V regions) from mRNAs of the obtained hybridomas using a reverse transcriptase, and linking them to DNAs encoding the desired antibody constant regions (C regions).

More specifically, without being particularly limited to the following examples, sensitizing antigens for obtaining the above-mentioned antibody genes encoding the H chains and L chains include both complete antigens with immunogenicity and incomplete antigens comprising haptens and such that do not show immunogenicity. For example, full-length proteins and partial peptides of proteins of interest can be used. In addition, it is known that substances composed of polysaccharides, nucleic acids, lipids and such may become antigens. Thus, there are no particular limitations on antigens of the antibodies of the present invention. Antigens can be prepared by methods known to those skilled in the art, and they can be prepared, for example, by the methods using baculoviruses (for example, see WO98/46777). Hybridomas can be produced, for example, by the method of Milstein *et al.* (G. Kohler and C. Milstein, Methods Enzymol. 1981, 73: 3-46). When the immunogenicity of an antigen is low, it can be linked to a macromolecule that has immunogenicity, such as albumin, and then used for immunization. Furthermore, by linking antigens to other molecules if necessary, they can be converted into soluble antigens. When transmembrane molecules such as receptors are used as antigens, portions of the extracellular regions of the receptors can be used as fragments, or cells expressing transmembrane molecules on their cell surface may be used as immunogens.

Antibody-producing cells can be obtained by immunizing animals using the suitable sensitizing antigens described above. Alternatively, antibody-producing cells can be prepared by *in vitro* immunization of lymphocytes that can produce antibodies. Various mammals can be used as the animals for immunization, where rodents, lagomorphas, and primates are generally used. Examples of such animals include mice, rats, and hamsters for rodents, rabbits for lagomorphas, and monkeys including cynomolgus monkeys, rhesus monkeys, hamadryas, and chimpanzees for primates. In addition, transgenic animals carrying human antibody gene repertoires are also known, and human antibodies can be obtained by using these animals (see WO96/34096; Mendez et al., Nat. Genet. 1997, 15: 146-56). Instead of using such transgenic animals, for example, desired human antibodies having binding activity to desired antigens can be obtained by sensitizing *in vitro* human lymphocytes with the desired antigens or cells expressing the desired antigens, and then fusing the sensitized lymphocytes with human myeloma cells such as U266 (see Japanese Patent Application Kokoku Publication No. HI-59878 (examined, approved Japanese patent application published for opposition)). Furthermore, desired human antibodies can be obtained by immunizing transgenic animals carrying a complete repertoire of human antibody genes, with desired antigens (see WO93/12227, WO92/03918, WO94/02602, WO96/34096, and WO96/33735).

Animal immunization can be carried out by appropriately diluting and suspending a sensitizing antigen in Phosphate-Buffered Saline (PBS), physiological saline or such, and forming an emulsion by mixing an adjuvant if necessary, followed by an intraperitoneal or subcutaneous injection into animals. After that, the sensitizing antigen mixed with Freund's incomplete adjuvant is preferably administered several times every four to 21 days. Antibody production can be confirmed by measuring the target antibody titer in animal sera using conventional methods.

Antibody-producing cells obtained from lymphocytes or animals immunized with a desired antigen can be fused with myeloma cells using conventional fusing agents (for example, polyethylene glycol) to generate hybridomas (Goding, Monoclonal Antibodies: Principles and Practice, Academic Press, 1986, 59-103). When required, hybridoma cells can be cultured and grown, and the binding specificity of the antibodies produced from these hybridomas can be measured using known analysis methods such as immunoprecipitation, radioimmunoassay (RIA), and enzyme-linked immunosorbent assay (ELISA). Thereafter, hybridomas that produce antibodies in which specificity, affinity, or activity of interest has been determined can be subcloned by methods such as limiting dilution.

Next, genes encoding the selected antibodies can be cloned from hybridomas or antibody-producing cells (sensitized lymphocytes and such) using probes that can specifically bind to the antibodies (for example, oligonucleotides complementary to sequences encoding the antibody constant regions). Cloning from mRNAs using RT-PCR is also possible. Immunoglobulins are classified into five different classes, IgA, IgD, IgE, IgG, and IgM. These classes are further divided into several subclasses (isotypes) (for example, IgG-1, IgG-2, IgG-3, and IgG-4; and IgA-1 and IgA-2). H chains and L chains used in the present invention to produce antibodies are not particularly limited and may derive from antibodies belonging to any of these classes or subclasses; however, IgGs are particularly preferred.

Herein, it is possible to modify H-chain-encoding genes and L-chain-encoding genes using genetic engineering techniques. Genetically modified antibodies such as chimeric antibodies and humanized antibodies that have been artificially modified to decrease heterologous antigenicity against humans, and such, can be appropriately produced if necessary for antibodies such as mouse antibodies, rat antibodies, rabbit antibodies, hamster antibodies, sheep antibodies, and camel antibodies. Chimeric antibodies are antibodies composed of H chain and L chain variable regions of a nonhuman mammal antibody such as mouse antibody, and the H chain and L chain constant regions of a human antibody. They can be obtained by linking DNAs encoding variable regions of a mouse antibody to DNAs encoding the constant regions of a human antibody, incorporating them into an expression vector, and introducing the vector into a host for production of the antibodies. A humanized antibody, which is also called a reshaped human antibody, can be obtained as follows: A DNA sequence designed to link the complementarity determining regions (CDRs) of an antibody of a nonhuman mammal such as a mouse is synthesized by PCR from a number of oligonucleotides produced so that they have overlapping portions at the ends of the sequence. The obtained DNA can be linked to a DNA encoding a human antibody constant region. The linked DNA can be incorporated into an expression vector, and the vector can be introduced into a host to produce the antibody (see EP239400 and WO96/02576). Human antibody FRs that are linked via the CDRs are selected so that the CDRs form a favorable antigen-binding site. If necessary, amino acids in the framework regions of antibody variable regions may be substituted such that the CDRs of a reshaped human antibody form an appropriate antigen-binding site (K. Sato et al., Cancer Res. 1993, 53: 851-856).

In addition to the humanization described above, antibodies may be modified to improve their biological properties, for example, binding activity to an antigen. Modifications in the present invention can be carried out using methods such as site-directed mutagenesis (see, for example, Kunkel (1985) Proc. Natl. Acad. Sci. USA 82: 488), PCR mutagenesis, and cassette mutagenesis. In general, mutant antibodies whose biological properties have been improved show amino acid sequence homology and/or similarity of 70% or higher, more preferably 80% or higher, and even more preferably 90% or higher (for example, 95% or higher, 97%, 98%, 99% and so on), when compared to the amino acid sequences of the original antibody variable regions. Herein, sequence homology and/or similarity is defined as the ratio of amino acid residues that are homologous (same residue) or similar (amino acid residues classified into the same group based on the general properties of amino acid side chains) to the original antibody residues, after the sequence homology value has been maximized by sequence alignment and gap introduction, if necessary. Generally, naturally-occurring amino acid residues are classified into groups based on the characteristics of their side chains: (1) hydrophobic: alanine, isoleucine, valine, methionine, and leucine; (2) neutral hydrophilic: asparagine, glutamine, cysteine, threonine, and serine; (3) acidic: aspartic acid and glutamic acid; (4) basic: arginine, histidine, and lysine; (5) residues that affect the orientation of the chain: glycine and proline; and (6) aromatic: tyrosine, tryptophan, and phenylalanine.

Ordinarily, a total of six complementarity determining regions (CDRs; hypervariable regions) present in the H chain and L chain variable regions interact to form the antigen binding site(s) of an antibody. Even one of these variable regions is known to have the ability to recognize and bind to the antigen, although the affinity will be lower than when all binding sites are included. Therefore, antibody genes of the present invention encoding H chains and L chains only have to encode fragment portions having each of the antigen binding sites of H chains and L chains, and polypeptides encoded by these genes only have to maintain binding activity to the desired antigens.

Heavy chain variable regions are ordinarily composed of three CDR regions and four FR regions as described above. In a preferred embodiment of the present invention, amino acid residues subjected to "modification" can be appropriately selected from among amino acid residues positioned in the CDR regions or FR regions. Generally, modification of the amino acid residues in the CDR regions can decrease the binding activity to antigens. Therefore, in the present invention, amino acid residues subjected to "modification" are not particularly limited but are preferred to be appropriately selected from among amino acid residues positioned in the FR regions. Even amino acid residues in the CDRs may be selected, as long as modifications of these residues have been confirmed not to reduce the binding activity.

Furthermore, sequences that can be used as variable region FRs of the antibodies of organisms such as humans or mice, can be appropriately obtained by those skilled in the art using public databases.

The present disclosure also relates to polypeptides comprising an FcRn-binding domain whose pharmacokinetics in blood is controlled by a method of the present invention.

In a preferred embodiment, the present disclosure provides humanized antibodies whose pharmacokinetics in blood is controlled by a method of the present invention. For example, the humanized antibodies are reshaped human antibodies comprising complementarity determining regions (CDRs) derived from a nonhuman animal, framework regions (FRs) derived from a human, and human constant regions, with at least one amino acid residue in the CDRs or FRs, which can be exposed on antibody surface, has a charge different from that of the corresponding amino acid residue in the wild-type CDRs or FRs, and whose variable regions are derived from an antibody of the nonhuman animal and phar-macokinetics in blood is controlled as compared to corresponding chimeric antibodies having the same constant regions.

The above-described "human constant region" preferably refers to a region comprising a wild-type human Fc domain; however, it may be a modified Fc.

The present invention also relates to methods for producing modified humanized IgG antibodies comprising an FcRn-binding domain, whose pharmacokinetics in blood is controlled by using the methods of the present invention. Specifically, the present invention provides methods for producing modified humanized IgG antibodies comprising an FcRn-binding domain, whose pharmacokinetics in blood is controlled. The present disclosure also includes modified humanized IgG antibodies comprising an FcRn-binding domain, which are produced by the methods of the present invention.

In a preferred embodiment, the production methods of the present invention are methods for producing modified humanized IgG antibodies comprising an FcRn-binding domain, having prolonged or reduced half-life in blood as compared to before said modification, and which comprise:
(a) modifying the nucleic acids encoding a humanized IgG antibody
   to change the charge of at least one amino acid residue that can be exposed on the surface of the polypeptides, wherein said at least one amino acid residue is an amino acid residue in a heavy chain or light chain variable region of said antibody, wherein the change in charge of said at least one amino acid residue Is achieved by amino acid substitution such that said modifying shifts the Isoelectric point of said antibody;
(b) culturing host cells to express the nucleic acids;
(c) collecting the modified antibody from the host cell cultures;
(d) analyzing the isoelectric point of said modified humanized IgG antibody;
(e) assessing the binding activity of said modified humanized IgG antibody; and
(f) assessing whether said half-life has been prolonged or reduced by conducting kinetic tests;
wherein the modified humanized IgG antibody retains its binding activity.

The phrase "modifying nucleic acids" in the above-mentioned methods of the present invention refers to modifying nucleic acids so that they correspond to amino acid residues introduced by the "modifications" of the present invention. More specifically, it refers to modifying the nucleic acids encoding the original (pre-modified) amino acid residues to the nucleic acids encoding the amino acid residues that are to be introduced by the modification. Ordinarily, it means performing a gene manipulation or a mutation treatment that would result in at least one nucleotide insertion, deletion, or substitution to an original nucleic acid so that codons encoding amino acid residues of interest is formed. More specifically, codons encoding the original amino acid residues are substituted with codons encoding the amino acid residues that are to be introduced by the modification. Such nucleic acid modifications can be suitably performed by those skilled in the art using known techniques such as site-directed mutagenesis and PCR mutagenesis.

Furthermore, nucleic acids of the present disclosure are usually carried by (inserted into) suitable vectors and then introduced into host cells. These vectors are not particularly limited so long as the inserted nucleic acids are stably maintained. For example, when using E. coli as the host, the cloning vector is preferably pBluescript vector (Stratagene) and such, but various commercially available vectors may be used. Expression vectors are particularly useful as vectors for producing the polypeptides of the present invention. Expression vectors are not particularly limited so long as they can express polypeptides in test tubes, E. *coli,* cultured cells, or individual organisms. For example, preferred vectors include pBEST vector (Promega) for expression in test tubes, pET vector (invitrogen) for E. *coli,* pME18S-FL3 vector (GenBank Accession No. AB009864) for cultured cells, and pME18S vector (Mol. Cell Biol. 8: 466-472 (1998)) for individual organisms. Insertion of a DNA of the present disclosure into vectors can be performed by standard methods such as ligase reactions using restriction enzyme sites (Current protocols in Molecular Biology edit. Ausubel et al. (1987) Publish. John Wiley & Sons. Section 11.4-11.11).

The above-mentioned host cells are not particularly limited, and various host cells can be used, depending on the purpose. Cells used for expressing the polypeptides include bacterial cells (for example, *Streptococcus, Staphylococcus, E. coli, Streptomyces,* and *Bacillus subtilis),* fungal cells (for example, yeast and *Aspergillus),* insect cells (for example, *Drosophila* S2 and *Spodoptera* SF9), animal cells (for example, CHO, COS, HeLa, C127, 3T3, BHK, HEK293, and Bowes melanoma cell), and plant cells. Vectors can be introduced into host cells using known methods such as the calcium phosphate precipitation method, electroporation method (Current protocols in Molecular Biology edit. Ausubel et al. (1987) Publish. John Wiley & Sons. Section 9.1-9.9), lipofection method, and microinjection method.

For secreting host cell-expressed polypeptides into the lumen of the endoplasmic reticulum, periplasmic space, or extracellular environment, suitable secretion signals can be incorporated into the polypeptides of interest. These signals may be intrinsic or foreign to the polypeptides of interest.

When the polypeptides of the present invention are secreted into the culture media, the polypeptides produced by the above-mentioned methods can be harvested by collecting the media. When the polypeptides of the present invention are produced inside cells, first, the cells are lysed, and then these polypeptides are collected.

The polypeptides of the present invention can be collected and purified from recombinant cell cultures by using known methods, including ammonium sulfate or ethanol precipitation, acidic extraction, anion or cation exchange chromatography, phosphocellulose chromatography, hydrophobic interaction chromatography, affinity chromatography, hydroxyapatite chromatography, and lectin chromatography.

The present disclosure relates to compositions (pharmaceutical agents) which comprise humanized IgG antibodies comprising an FcRn-binding domain, whose pharmacokinetics in blood is controlled by the methods of the present invention, and pharmaceutically acceptable carriers.

In the present disclosure, pharmaceutical compositions usually refer to pharmaceutical agents for treating or preventing, or testing and diagnosing diseases.

The pharmaceutical compositions of the present disclosure can be formulated by methods known to those skilled in the art. For example, such pharmaceutical compositions can be used parenterally, as injections which are sterile solutions or suspensions including the compositions along with water or another pharmaceutically acceptable liquid. For example, such compositions may be formulated as unit doses that meet the requirements for the preparation of phar-maceuticals by appropriately combining the compositions with pharmaceutically acceptable carriers or media, specifically with sterile water, physiological saline, a vegetable oil, emulsifier, suspension, surfactant, stabilizer, flavoring agent, excipient, vehicle, preservative, binder or such. In such preparations, the amount of active ingredient is adjusted such that an appropriate dose that falls within a pre-determined range can be obtained.

Sterile compositions for injection can be formulated using vehicles such as distilled water for injection, according to standard protocols for formulation.

Aqueous solutions for injection include, for example, physiological saline and isotonic solutions containing dextrose or other adjuvants (for example, D-sorbitol, D-mannose, D-mannitol, and sodium chloride). Appropriate solubilizers, for example, alcohols (ethanol and such), polyalcohols (propylene glycol, polyethylene glycol, and such), nonionic surfactants (polysorbate 80^{™}, HCO-50, and such), may be used in combination.

Oils include sesame and soybean oils. Benzyl benzoate and/or benzyl alcohol can be used in combination as solubilizers. Buffers (for example, phosphate buffer and sodium acetate buffer), soothing agents (for example, procaine hydrochloride), stabilizers (for example, benzyl alcohol and phenol), and/or antioxidants can also be combined. Prepared injections are generally filled into appropriate ampules.

The pharmaceutical compositions of the present disclosure are preferably administered parenterally. For exam-ple, the compositions may be injections, transnasal compositions, transpulmonary compositions or transdermal compo-sitions. For example, such compositions can be administered systemically or locally by intravenous injection, intramus-cular injection, intraperitoneal injection, subcutaneous injection or such.

The administration methods can be appropriately selected considering the patient's age and symptoms. The dose of a pharmaceutical composition comprising an antibody or a polynucleotide encoding an antibody may be, for example, from 0.0001 to 1000 mg/kg for each administration. Alternatively, the dose may be, for example, from 0.001 to 100,000 mg per patient. However, the doses in the present disclosure are not limited to the ranges described above. The doses and administration methods vary depending on a patient's weight, age, symptoms and such. Those skilled in the art can select appropriate doses and administration methods considering the factors described above.

The present disclosure also provides nucleic acids encoding polypeptides that comprise humanized antibodies comprising an FcRn-binding domain, whose pharmacokinetics in blood is controlled by the methods of the present invention. Furthermore, vectors carrying these nucleic acids are encompassed by the present disclosure.

The present disclosure also provides host cells carrying the above described nucleic acids. The host cells are not particularly limited and include, for example, *E. coli* and various animal cells. The host cells may be used, for example, as a production system to produce and express the antibodies of the present invention. *In vitro* and *in vivo* production systems are available for polypeptide production systems. Production systems that use eukaryotic cells or prokaryotic cells are examples of *in vitro* production systems.
▪ Eukaryotic cells that can be used as a host cell include, for example, animal cells, plant cells, and fungal cells. Animal cells include mammalian cells such as CHO (J. Exp. Med. (1995) 108: 945), COS, HEK293, 3T3, myeloma, BHK (baby hamster kidney), HeLa, and Vero; amphibian cells such as *Xenopus laevis* oocytes (Valle, et al. (1981) Nature 291: 338-340); and insect cells such as Sf9, Sf21, and Tn5. In the expression of the antibodies of the present invention, CHO-DG44, CHO-DX11B, COS7 cells, HEK293 cells, and BHK cells can be suitably used. Among animal cells, CHO cells are particularly preferable for large-scale expression. Vectors can be introduced into a host cell by, for example, calcium phosphate methods, DEAE-dextran methods, methods using cationic liposome DOTAP (Boehringer-Mannheim), electroporation methods, or lipofection methods.

Plant cells include, for example, *Nicotiana* tabacum-derived cells and duckweed *(Lemna minor)* known as a protein production system. Calluses can be cultured from these cells to produce the antibodies of the present invention. Known protein production systems are those using fungal cells including yeast cells, for example, cells of genus Sac*charomyces* (such as *Saccharomyces cerevisiae* and *Saccharomyces pombe);* and cells of filamentous fungi, for example, genus *Aspergillus* (such as *Aspergillus niger).* These cells can be used as a host to produce the antibodies of the present invention.

Bacterial cells can be used in prokaryotic production systems. Examples of bacterial cells include *Bacillus subtilis* as well as E. *coli* described above. Such cells can be used to produce the antibodies of the present invention.

When producing an antibody using a host cell of the present disclosure, the polynucleotide encoding an antibody of the present invention may be expressed by culturing the host cell transformed with an expression vector containing the polynucleotide. The culture can be performed using known methods. For example, when using animal cells as a host, DMEM, MEM, RPMI 1640, or IMDM may be used as the culture medium, and may be used with or without serum supplements such as FBS or fetal calf serum (FCS). The preferred pH is about 6 to 8 during the culture. The culture is carried out typically at a temperature of about 30 to 40°C for about 15 to 200 hours. Medium is exchanged, aerated, or agitated, as necessary.

On the other hand, production systems using animals or plants may be used as systems for producing polypeptides *in vivo.* For example, a polynucleotide of interest is introduced into an animal or plant and the polypeptide is produced in the body of the animal or plant and then collected. The "hosts" of the present invention include such animals and plants.

Animals to be used for production systems include mammals and insects. Mammals such as goats, pigs, sheep, mice, and cattle may be used (Vicki Glaser SPECTRUM Biotechnology Applications (1993)). Alternatively, the mammals may be transgenic animals.

For example, a polynucleotide encoding an antibody of the present invention may be prepared as a fusion gene with a gene encoding a polypeptide specifically produced in milk, such as the goat β-casein gene. Polynucleotide fragments containing the fusion gene are injected into goat embryos, which are then transplanted into female goats. The desired antibody can be obtained from milk produced by the transgenic goats born from the goats that received the embryos, or from their offspring. Appropriate hormones may be administered to the transgenic goats to increase the volume of milk containing the antibody produced by the goats (Ebert et al., Bio/Technology 12: 699-702 (1994)).

Insects such as silkworms, may also be used for producing the antibodies of the present invention. Baculoviruses carrying a polynucleotide encoding an antibody of interest can be used to infect silkworms, and the antibody of interest can be obtained from the body fluids of the silkworms (Susumu et al., Nature 315: 592-594 (1985)).

Plants used for producing the antibodies of the present invention include, for example, tobacco. When tobacco is used, a polynucleotide encoding an antibody of interest is inserted into a plant expression vector, for example, pMON 530, and then the vector is introduced into a bacterium such as *Agrobacterium tumefaciens.* The bacteria are then used to infect tobacco such as *Nicotiana tabacum,* and the desired antibody can be obtained from the leaves (Ma et al., Eur. J. Immunol. 24: 131-138 (1994)). Alternatively, duckweed *(Lemna minor)* is infected with similar bacteria and then cloned. The desired antibody can be obtained from the cloned duckweed cells (Cox KM et al. Nat. Biotechnol. 2006 Dec; 24(12): 1591-1597).

The resulting antibody may be isolated from the inside or outside (such as the medium and milk) of host cells, and purified as a substantially pure and homogenous antibody. Methods for isolating and purifying antibodies are not limited to any specific method and any standard method for isolating and purifying polypeptides may be used. Antibodies can be isolated and purified, by appropriately selecting or combining, for example, chromatographic columns, filtration, ultrafiltration, salting out, solvent precipitation, solvent extraction, distillation, immunoprecipitation, SDS-polyacrylamide gel electrophoresis, isoelectric focusing, dialysis, recrystallization, and others.

Chromatographies include, for example, affinity chromatographies, ion exchange chromatographies, hydrophobic chromatographies, gel filtrations, reverse-phase chromatographies, and adsorption chromatographies (Strategies for Protein Purification and Characterization: A Laboratory Course Manual. Ed Daniel R. Marshak et al., Cold Spring Harbor Laboratory Press, 1996). These chromatographies can be carried out using liquid phase chromatographies such as HPLC and FPLC. Examples of affinity chromatography columns include protein A columns and protein G columns. Examples of the proteins A columns include Hyper D, POROS, and Sepharose F. F. (Pharmacia).

An antibody can be modified freely and peptide portions can be deleted from it by treating the antibody with an appropriate protein modifying enzyme before or after antibody purification, as necessary. Such protein modifying enzymes include, for example, trypsins, chymotrypsins, lysyl endopeptidases, protein kinases, and glucosidases.

The above-described methods for producing modified humanized IgG antibodies of the present invention comprising an FcRn-binding domain, whose pharmacokinetics in blood is controlled, which comprise culturing host cells of the present invention and collecting the polypeptides from the cell cultures, are also preferred embodiments of the present invention.

### Examples

Herein below, the present invention is specifically described with reference to Examples, but it is not to be construed as being limited thereto.

### [Example 1] Humanization of bispecific antibody

A bispecific antibody consisting of the combination of the anti-Factor IXa antibody A69-VH, anti-Factor X antibody B26-VH, and hybrid L chain (BBA), which was found to be most effective in shortening blood coagulation time in Japanese Patent Application No. 2005-112514, was humanized as described below.

### 1-1. Homology search of humanized antibodies

Using a database constructed by obtaining amino acid sequence data of human antibodies from the publicly-disclosed Kabat Database (ftp://ftp.ebi.ac.uk/pub/databases/kabat!) and IMGT Database (http://imgt.cines.fr/), homology search was carried out separately for the mouse A69-H chain variable region (amino acid sequence; SEQ ID NO: 15), mouse B26-H chain variable region (amino acid sequence; SEQ ID NO: 16), and mouse BBA-L chain variable region (amino acid sequence; SEQ ID NO: 17). The results confirmed that they have high homologies to the human antibody sequences shown below, and thus it was decided that they would be used as the framework regions (hereinafter abbreviated as FRs) of humanized antibodies.
(1)A69-H chain variable region: KABATID-000064 (Kabat Database)
   (Kipps et al., J. Clin. Invest. 1991; 87:2087-2096)
(2)B26-H chain variable region: EMBL Accession No. AB063872 (IMGT Database)
   (Unpublished data)
(3) BBA-L chain variable region: KABATID-024300 (Kabat Database)
   (Welschof et al., J. Immunol. Method 1995; 179:203-214)

Humanized antibodies in which complementarity determining regions (hereinafter abbreviated as CDRs) of each mouse antibody were grafted into the FRs of the human antibodies (1)-(3) were prepared.

Also, the web homology search site publicly disclosed by NCBI (http://www.ncbi.nlm.nih.gov/BLAST/) was used to search secretory signal sequences of human antibodies that are highly homologous to the human antibodies of (4)-(6). The following secretory signal sequences obtained by the search were used.
(4) A69-H chain variable region: GenBank Accession No. AF062257
(5) B26-H chain variable region: GenBank Accession No. AAC18248
(6) BBA-L chain variable region: GenBank Accession No. AAA59100

### 1-2. Construction of humanized antibody gene expression vector

Twelve synthetic oligoDNAs of about 50 bases were prepared from a nucleotide sequence encoding the amino acid sequence from a secretory signal sequence to an antibody variable region, such that about 20 bases of their 3'-end anneal with each other. The synthetic oligoDNAs were designed so that the 5'-terminal nucleotides encode a human sequence, the 3'-terminal nucleotides encode a mouse sequence, or all nucleotides encode human sequences. Furthermore, a primer annealing to the 5'-end of an antibody variable region gene and having the Xhol cleavage sequence, and a primer annealing to the 3'-end of an antibody variable region gene, having the Sfil cleavage sequence and also encoding the 5'-end sequence of an intron sequence were prepared.

1 µl each of the synthetic oligoDNAs prepared at 2.5 µM were mixed, and 1x TaKaRa Ex Taq Buffer, 0.4 mM dNTPs, and 0.5 units TaKaRa Ex Taq (all from Takara) were added to prepare 48 µl of a reaction solution. After heating this at 94°C for 5 minutes, 2 cycles of reacting at 94°C for 2 minutes, 55°C for 2 minutes, and 72°C for 2 minutes were performed to assemble and elongate each of the synthetic oligoDNAs. Next, 1 µl (10 µM each) of primers annealing to the 5'-end and to the 3'-end of the antibody gene were added, and the antibody variable region genes were amplified by 35 cycles of reacting at 94°C for 30 seconds, 55°C for 30 seconds, and 72°C for 1 min and then reacting at 72°C for 5 minutes. After PCR, the whole amount of the reaction solution was subjected to 1% agarose gel electrophoresis. Amplified fragments having the size of interest (approximately 400 bp) were purified using the QlAquick Gel Extraction Kit (QIAGEN) according to the method described in the instruction manual, and were eluted with 30 µl of sterile water. These fragments were cloned using the pGEM-T Easy Vector System (Promega) according to the method described in the instruction manual. The nucleotide sequence of each of the DNA fragments was determined using the BigDye Terminator Cycle Sequencing Kit (Applied Biosystems) and ABI PRISM 3730xL DNA Sequencer or ABI PRISM 3700 DNA Sequencer (Applied Biosystems) according to the method described in the instruction manual.

The H-chain variable region fragment-inserted plasmid and the L-chain variable region fragment-inserted plasmid, each of which was confirmed to have a correct humanized antibody variable region gene sequence, were digested with Xhol and Sfil, and EcoRI respectively. Next, the reaction solution was subjected to 1% agarose gel electrophoresis. DNA fragments having the size of interest (approximately 400 bp) were purified using QlAquick Gel Extraction Kit (QIAGEN) according to the method described in the instruction manual, and eluted with 30 µl of sterile water. Then, expression vectors for animal cells were prepared as follows. To preferentially express IgG4 whose H chains are of a heterologous combination, a CH3 portion amino acid-substituted IgG4 was used by referring to the knobs-into-holes technique of IgG1 (Merchant AM et al., Nature Biotechnology, Vol.16, p.677-681 (1998)). Furthermore, to promote H chain dimer formation, amino acid substitution (-ppcpScp- → -ppcpPcp-) was also introduced to the hinge. Humanized A69 H chain expression vector was prepared by inserting a humanized A69 H chain variable region antibody gene fragment into an expression vector prepared by inserting Y349C and T366W-substituted constant region gene to pCAGGS comprising a chicken β-actin promoter (Niwa et al., Gene Vol.108, p.193-199 (1991)). In addition, humanized B26 H chain expression vector was prepared by inserting a humanized B26 H chain variable region antibody gene fragment into an expression vector prepared by inserting E356C, T366S, L368A, and Y407V-substituted constant region gene to pCAGGS. The plasmid (pCAG-gκDNA) prepared by inserting a wild type antibody L chain constant region into pCAGGS was digested with EcoRI to prepare an expression vector inserted with a humanized BBA L chain variable region antibody gene fragment. Ligation reaction was performed using Rapid DNA Ligation Kit (Roche Diagnostics), and DH5α strain *E*. *coli* (TOYOBO) was transformed.

### 1-3. Expression of the humanized bispecific antibody

The humanized bispecific antibody was expressed according to the following method. Human fetal renal carcinoma cell-derived HEK293H strain (Invitrogen) was suspended in DMEM medium (Invitrogen) containing 10% Fetal Bovine Serum (Invitrogen), and 10 ml of this was seeded at a cell density of 5-6 × 10⁵ cells/ml in each dish used for adhesive cells (10-cm diameter, CORNING) and cultured for one day in a CO₂ incubator (37°C, 5% CO₂), Then, the medium was removed by suction, and 6.9 ml of CHO-S-SFM-II medium (Invitrogen) containing 1% Fetal Bovine Serum (Invitrogen) was added. The plasmid DNA mixture solution prepared in Example 1-2 (total of 13.8 µg) was mixed with 20.7 µl of 1 µg/ml Polyethylenimine (Polysciences Inc.) and 690 µl of CHO-S-SFMII medium, left to stand at room temperature for 10 minutes, then the cells were added to the cells in each dish and incubated in a CO₂ incubator (37°C, 5% CO₂) for 4-5 hours. Thereafter, 6.9 ml of CHO-S-SFM-II medium (Invitrogen) containing 1% Fetal Bovine Serum (Invitrogen) was added and then the cells were incubated in a CO₂ incubator for 3 days. The culture supernatant was recovered, then cells were removed by centrifugation (at approximately 2000 g for 5 minutes at room temperature), and the solution was sterilized by passing it through a 0.22 µm filter MILLEX^{®}-GV (Millipore). The sample was stored at 4°C until use.

### 1-4. Purification of the humanized bispecific antibody

100 µl of rProtein A Sepharose^{™} Fast Flow (Amersham Biosciences) was added to the culture supernatant obtained according to the method described in Example 1-2, and the solution was mixed by inversion at 4°C for 4 hours or more. The solution was transferred to a 0.22 µm filter cup, Ultrafree^{®}-MC (Millipore), and after washing 3 times with 500 µl of TBS containing 0.01% Tween^{®} 20, the rProtein A Sepharose^{™} resin was suspended in 100 µl of 50 mM sodium acetate solution containing 0.01% Tween^{®} 20 at pH 3.3 and left to stand for 2 minutes. Then, the antibody was eluted, and the eluate was immediately neutralized by adding 6.7 µl of 1.5 M Tris-HCl, pH 7.8.

### 1-5. Quantification of humanized bispecific antibody concentration

The antibody concentration was determined using the following two types of methods.

Goat anti-human IgG (Biosource International) was adjusted to 1 µg/ml with a coating buffer, and immobilized to a Nunc-Immuno plate (Nunc). After blocking with a diluent buffer (D.B.), a sample of the culture supernatant suitably diluted with D.B. was added. Furthermore, as a standard for calculating the antibody concentration, human IgG4 (humanized anti-TF antibody, see WO 99/51743) diluted with D.B. in a three-fold dilution series up to eleven stages starting from 2000 ng/ml was added similarly. After three washes, a goat anti-human IgG, alkaline phosphatase (Biosource International) was reacted. After five washes, the color was developed using Sigma 104^{®} phosphatase substrate (Sigma-Aldrich) as a substrate, and the absorbance at 405 nm was measured on an absorbance reader Model 3550 (Bio-Rad Laboratories) with a reference wavelength of 655 nm. Using the Microplate Manager III (Bio-Rad Laboratories) software, human IgG concentration in the culture supernatant was calculated from the standard curve.

Furthermore, the antibody concentration was quantified using Sensor Chip CM5 (BIACORE) to which Protein A had been immobilized, with Biacore 1000 or Biacore Q (BIACORE). More specifically, Protein A-immobilized sensor chip was prepared according to the manufacturer's protocol by reacting an activated sensor chip with a Protein A solution (SIGMA) diluted to 50 µg/ml with 10 mM aqueous sodium acetate solution (pH 4.0, BIACORE) at 5 µl/min for 30 minutes, and then performing a blocking operation. This sensor chip was used to measure the concentration of the culture supernatant and the purified product using BIAcore 1000 (BIACORE). HBS-EP Buffer (BIACORE) was used for the immobilization of the sensor chip and for the measurements of concentration. As a standard for concentration measurements, humanized IgG4 antibody (humanized anti-tissue factor antibody, see WO 99/51743) diluted with HBS-EP Buffer in a two-fold dilution series up to six stages beginning at 4000 ng/ml was used.

### 1-6. Assessment of blood coagulation activity of humanized bispecific antibody

To elucidate whether a bispecific antibody corrects the coagulation ability of hemophilia A blood, effects of the bispecific antibody on activated partial thromboplastin time (APTT) were examined using Factor VIII-deficient plasma. A mixed solution comprising 50 µl of an antibody solution at various concentrations, 50 µl of Factor VIII-deficient plasma (Biomerieux), and 50 µl of APTT reagent (Dade Behring) was heated at 37°C for 3 minutes. Coagulation reaction was initiated by adding 50 µl of 20 mM CaCl₂ (Dade Behring) to this mixed solution. The time required for coagulation was measured with CR-A (Amelung)-connected KC10A (Amelung).

Using a calibration curve provided by defining the coagulation time of Factor VIII-deficient plasma as 0% and that of normal plasma as 100%, Factor VIII-like activity (%) of a bispecific antibody was calculated from the coagulation time measured when the bispecific antibody was added.

### 1-7. Preparation of humanized bispecific antibody retaining blood coagulation activity

For humanized bispecific antibodies which had reduced blood coagulation ability in the above-described blood coagulation activity assessment, amino acids of their human antibody FRs were modified to increase their activities. Specifically, mutations were introduced to the humanized antibody variable region using a QuikChange Site-Directed Mutagenesis Kit (Stratagene) according to the method described in the instruction manual. The H-chain variable region fragment-inserted plasmid and L-chain variable region fragment-inserted plasmid were confirmed to have the humanized antibody variable region gene sequences of interest and were digested with Xhol and Sfil, and EcoRI respectively. The reaction solution was subjected to 1% agarose gel electrophoresis. DNA fragments having the size of interest (approximately 400 bp) were purified using a QIAquick Gel Extraction Kit (QIAGEN) according to the method described in the instruction manual, and eluted with 30 µl of sterile water. Then, expression vectors for animal cells were prepared according to the method described in Example 1-2. A humanized bispecific antibody was prepared according to the method described in Examples 1-3, 1-4, and 1-5 and blood coagulation activity was evaluated according to the method described in Example 1-6.

By repeated amino acid modifications of the FR sequences and assessment of blood coagulation ability, a humanized bispecific antibody (humanized A69 (hA69a)/humanized B26 (hB26-F123e4)/humanized BBA (hAL-F123j4)) having the same level of activity as the chimeric bispecific antibody (A69/B26/BBA) was obtained (Fig. 1). Each of the antibody variable region sequences is indicated in the following SEQ ID NOs.
(1) humanized A69 antibody VH (hA69a) SEQ ID NO: 1 (nucleotide sequence), SEQ ID NO: 2 (amino acid sequence)
(2) humanized B26 antibody VH (hB26-F123e4) SEQ ID NO: 3 (nucleotide sequence), SEQ ID NO: 4 (amino acid sequence)
(3) humanized BBA antibody VL (hAL-F123j4) SEQ ID NO: 5 (nucleotide sequence), SEQ ID NO: 6 (amino acid sequence)

### [Example 2] Selection of amino acid modification sites in variable regions to isolate a bispecific antibody

Antibody Fv region models for humanized A69 and B26 antibodies were prepared by homology modeling using MOE software (Chemical Computing Group Inc.) to confirm amino acid residues exposed on the surface of variable regions of these antibodies. The models are shown in Fig. 2. A detailed analysis of the models suggested that among surface-exposed amino acids in the FR sequences other than CDRs, those at H10, H12, H23, H39, H43, and H105 (according to the Kabat's numbering; Kabat EA et al. 1991. Sequences of Proteins of Immunological Interest. NIH) were candidate amino acids that could be modified to shift the isoelectric point without reduction of activity. The amino acid at H97 was selected as a surface-exposed amino acid in CDRs.

### [Example 3] Modification of variable region amino acid sequences of humanized A69 antibody and modified form thereof, and humanized B26 antibody

Amino acids of the H chain variable regions of humanized A69 and B26 antibodies were modified to shift the isoelectric points of the antibodies. Specifically, mutations were introduced into the H chain variable regions of humanized A69 antibody (hA69a; nucleotide SEQ ID NO: 1) and humanized B26 antibody (hB26-F123e4; nucleotide SEQ ID NO: 3) prepared using a QuikChange Site-Directed Mutagenesis Kit (Stratagene) according to the method described in the attached instruction manual. H chain variable region fragment-inserted plasmids which had been confirmed to have the sequence of humanized antibody variable region gene of interest were digested with Xhol and Sfil, and then the reaction mixtures were subjected to electrophoresis using 1% agarose gel. DNA fragments having the size of interest (about 400 bp) were purified using a QIAquick Gel Extraction Kit (QIAGEN) according to the method described in the attached instruction manual, and then eluted using 30 µl of sterile water. The DNA fragments were inserted into an expression plasmid carrying the wild type constant region by the method described in Example 1-2 to construct H chain expression vectors. Modified amino acid residues in the respective antibodies and their SEQ IDs are shown in Table 1. (hA69-N97R, hA69-p18), and humanized B26 antibody (hB26-F123e4) and its modified form (hB26-p15) were prepared. A humanized A69 antibody (hA69a) and its modified forms (hA69-N97R, hA69-p18) were expressed using a combination of H chain expression vectors (variable region is hA69-N97R, hA69-p18) and an an L chain expression vector (variable region is hAL-F123j4; SEQ ID NO: 6) according to Example 1-3. Meanwhile, a humanized B26 antibody (hB26-F123e4) and its modified form (hB26-p15) were expressed using a combination of H chain expression vectors (variable region is hB26-F123e4, hB26-p15) and an L chain expression vector (variable region is B26-VL; the amino acid sequence is described in WO2005/035756 (SEQ ID NO: 18)) according to Example 1-3. The antibodies were purified from culture supernatants by the method described in Example 1-4.

**Table 1**

| Name | Humanized A69-H chain variable region | |
|---|---|---|
| | Modification site | Amino acid SEQ ID NO: |
| hA69a | - | 2 |
| hA69-p18 | Q43E, Q105E | 7 |
| hA69-N97R | N97R | 9 |
| | | |

| Name | Humanized B26-H chain variable region | |
|---|---|---|
| | Modification site | Amino acid SEQ ID NO: |
| hB26-F123e4 | - | 4 |
| hB26-p15 | Q39K, Q43K, Q105R | 10 |

### [Example 4] Establishment of a cell line that expresses a bispecific antibody derived from humanized A69 or B26 antibodies

To prepare a humanized bispecific antibody, an antibody-expressing cell line was established by the procedure described below.

An H-chain constant region was amplified by PCR using a wild-type human IgG4 H-chain constant region gene as a template and using a 5'-end primer designed so that the nucleotide sequence encoding two amino acids (Ala-Ser) in the N-terminal side of the H-chain constant region will be an Nhel recognition sequence (GCTAGC) and a primer that anneals to the 3'-end and that carries a NotI recognizing site. Then, the amplified fragments were ligated to pBluescriptKS+ vector (TOYOBO) digested with Nhel and Not I (both from Takara) to prepare pBCH4 (comprising an IgG4 constant region gene). PCR was performed using primers which are complementary to the 5'-end nucleotide sequence of the H-chain variable regions of the humanized A69-H chain antibody (hA69-PFL: SEQ ID NO: 11) and humanized B26-H chain antibody (hB26-PF: SEQ ID NO: 12) and which have a Kozak sequence (CCACC) and an EcoRI recognition sequence, and a primer on the 3'-end nucleotide sequence having an Nhel recognition sequence. The obtained PCR products were digested with EcoRI and Nhel (both from Takara) and inserted into pBCH4 also digested with EcoRI and Nhel, and then the variable regions and the constant regions were linked. The prepared vector for humanized A69-H chain antibody was digested with EcoRI and Notl (both Takara), and then cloned into the animal cell expression vector pCXND3 digested with EcoRI and NotI. The procedure for the construction of the vector pCXND3 is described below.

DHFR-ΔE-rVH-PM1-f (see WO92/19759) was cleaved at the EcoRI and SmaI restriction sites to separate the vector backbone form the antibody H chain gene. Only the vector backbone was recovered, and then an EcoRI-Notl-BamHI adaptor (Takara) was cloned into it. The resulting vector was named pCHOI. The DHFR gene expression region derived from pCHOI was cloned into pCXN (Niwa et al., Gene 108: 193-200 (1991)) at the Hindlll restriction site. The resulting vector was named pCXND3. In addition, the prepared vector for humanized B26-H chain antibody was digested with EcoRI and Notl (both Takara), and then cloned into the animal cell expression vector pCXZD1 digested with EcoRI and Notl. pCXZD1 vector is an expression vector obtained from pCXND3 by substituting the Zeocin resistance gene for the neomycin resistance gene. Furthermore, an L-chain expression vector was prepared by inserting the L chain variable region of the humanized BBA-L chain antibody (hAL-s8; SEQ ID NO: 8) into a plasmid (pCAG-gκDNA) having an inserted L chain constant region according to Example 1-2. The prepared three types of expression vectors were linearized with restriction enzymes and then introduced into CHO-DG44 cells to establish an antibody-expressing cell line.

A stable expression cell line was prepared by the procedure described below. Genes were introduced by electroporation using GenePulserXcell (Bio-Rad). Each antibody expression vector was combined with 0.75 ml of CHO cells suspended in PBS (1 × 10⁷ cells/ml). The mixtures were cooled on ice for ten minutes, transferred into cuvettes, and pulsed at 1.5 kV and 25 µFD. After a ten-minute restoration period at room temperature, the electroporated cells were suspended in 40 ml of CHO-S-SFMII medium (Invitrogen) containing 1x HT supplement (Invitrogen). The suspension was diluted 10 times with the same medium and aliquoted into 96-well culture plates at 100 µl/well. After 24 hours of culture in a CO₂ incubator (5% CO₂), Geneticin and Zeocin (both Invitrogen) were added at 0.5 mg/ml and 0.6 mg/ml, respectively. The cells were cultured for two weeks. Expansion cultures were sequentially performed for colonies of drug-resistant transformants. An established high expression cell line was used for large scale culture to obtain the culture supernatant.

### [Example 5] Separation and purification of humanized antibody homodimers and a humanized bispecific antibody

Using the method described below, the bispecific antibody was purified from the culture supernatants obtained in Example 4. The culture supernatants were loaded onto an rProtein A Sepharose Fast Flow column (Amersham Biosciences; 50 mm I.D. x 9.9 cm H. = 194.3 ml resin) equilibrated with an equilibration buffer (20 mmol/l sodium phosphate buffer, 150 mol/l NaCl, pH 7.0). After washing with wash buffer 1 (20 mmol/l sodium phosphate buffer, 150 mol/l NaCl, pH 7.0) and wash buffer 2 (50 mmol/l sodium acetate buffer, pH 6.0), the column was eluted with 50 mmol/l acetic acid. Immediately after elution, pH was adjusted to 6.3 by adding 1.5 mol/l Tris-HCl (pH 7.8).

The resulting purified solution was loaded onto an SP TOYOPEARL 650M column (Tosoh; 26 mm I.D. x 22.3 cm H. = 118.3 ml resin) equilibrated with Solvent A (10 mmol/l sodium phosphate buffer, pH 6.3). The antibodies were separated based on their surface charges using the solutions and gradients indicated below.
Solvent A: 20 mmol/l sodium acetate buffer (pH 6.0)
Solvent B: 20 mmol/l sodium acetate buffer, 1 mol/l NaCl (pH 6.0)
Flow rate: 10 ml/min (113 cm/h); 5.3 ml/min (60 cm/h) only at the time of elution

| | | | |
|---|---|---|---|
| Gradient: | 0 → 15% B | stepwise | 3 column volumes (CV) |
| | 15 → 35% B | gradient | 6 CV |
| | 35 → 50% B | gradient | 10 CV |
| | 50 → 100% B | gradient | 3 CV |
| | 100% B | stepwise | 4 CV |

Two types of homodimers (hA69-PF and hB26-PF) and a single type of heterodimer, the bispecific antibody BiAb, were obtained by collecting eluted fractions of the detected three peaks separately.

### [Example 6] Analysis of prepared antibodies by isoelectric focusing

ATF is a previously obtained monoclonal antibody against human tissue factor, and is a humanized antibody comprising the constant region of human IgG4. The origin of ATF is described in detail in WO99/051743. The amino acid sequences of its H chain and L chain variable regions are shown in SEQ ID Nos: 13 and 14, respectively. hA69-PF, BiAb, and hB26-PF prepared in Example 5; hA69-N97R, hA69-p18, hB26-e, and hB26-p15 prepared in Example 3; and ATF were analyzed by isoelectric focusing to assess changes in the surface charge due to the following: differences in the amino acid sequences of their variable regions and amino acid modifications.

ATF, hA69-PF, BiAb, hB26-PF, and the humanized A69 antibody hA69-N97R and a modified form thereof, hA69-p18, as well as the humanized B26 antibody hB26-F123e4 and a modified form thereof, hB26-p15, were subjected to isoelectric focusing, as described below. Using Phastsystem Cassette (Amersham Bioscience), Phast-Gel Dry IEF gel (Amersham Bioscience) was allowed to swell for about 30 minutes in the swelling solution indicated below.

| | |
|---|---|
| Milli-Q water | 1.5 ml |
| Pharmalyte 5-8 for IEF (Amersham Bioscience) | 50 µl |
| Pharmalyte 8-10.5 for IEF (Amersham Bioscience) | 50 µl |

Electrophoresis was carried out in PhastSystem (Amersham Bioscience) using the swollen gel according to the program indicated below. The samples were loaded onto the gel in Step 2. The pI marker used is a calibration kit for pI (Amersham Bioscience).

| | | | | | |
|---|---|---|---|---|---|
| Step 1: | 2000 V | 2.5 mA | 3.5 W | 15°C | 75 Vh |
| Step 2: | 200 V | 2.5 mA | 3.5 W | 15°C | 15 Vh |
| Step 3: | 2000 V | 2.5 mA | 3.5 W | 15°C | 410 Vh |

After electrophoresis, the gel was fixed with 20% TCA, and then silver-stained using a silver staining kit protein (Amersham Bioscience) according to the protocol attached to the kit. After staining, the pls of the samples were calculated from the known pIs of the pI marker. The analysis result of isoelectric focusing is shown in Fig. 3. The calibration curve of pI vs mobility prepared using the pI marker and pls calculated from the curve are shown in Fig. 4. The pls were calculated based on the mobility of major bands since each sample exhibited antibody-derived charge heterogeneity.

The result showed that the surface charges were changed due to differences in the amino acid sequences of the variable regions and that the pls were shifted due to the change in surface charge through amino acid modifications. The pls were as follows: about 9.2 for hB26-PF, about 8.7 for BiAb, about 8.0 for hA69-PF, about 7.2 for ATF, about 8.9 for hA69-N97R, about 8.5 for hA69-p18, about 8.7 for hB26-F123e4, and about 9.0 for hB26-p15, hA69-N97R, hA69-p18, and hA69-PF were obtained by modifying the same humanized antibody variable region. A pI shift of about 0.9 could be achieved in hA69-PF compared with hA69-N97R, and a pI shift of about 0.3 could be achieved in hB26-p15 compared with hB26-F123e4. The examination described above demonstrates that pI can be shifted depending on the amino acid sequence of a variable region as well as by modifying a surface amino acid at H10, H12, H23, H39, H43, H97, or H105 in a selected variable region to change its charge.

### [Example 7] Assessment of humanized antibodies A69 and B26 and modified forms thereof for their binding activity

The functions of the humanized A69 antibody and its modified form were assessed by assaying their binding activities to the antigen Factor IXa, as described below. Humanized A69 antibody (hA69a) and its modified form, hA69-N97R, were assessed by the following procedure. Factor IXaβ (Enzyme Research Laboratories) diluted to 1 µg/ml with a coating buffer (100 mM sodium bicarbonate (pH 9.6), 0.02% sodium azide) was aliquoted (100 µl/well) into a Nunc-Immuno plate (a Nunc-Immuno^{™} 96 MicroWell^{™} MaxiSorp^{™} plate (Nalge Nunc International)), and then the plate was incubated overnight at 4°C. After washing three times with PBS(-) containing Tween^{(R)} 20, the plate was blocked with a diluent buffer (50 mM Tris-HCl (pH 8.1), 1% bovine serum albumin, 1 mM MgCl₂, 0.15 M NaCl, 0.05% Tween^{(R)} 20, 0.02% sodium azide) at room temperature for two hours. After removal of the buffer, the purified antibodies diluted with the diluent buffer were added to the plate at 100 µl/well. Then, the plate was incubated at room temperature for one hour. After the plate was washed three times, alkaline phosphatase-labeled goat anti-mouse IgG (BIOSOURCE) diluted to 1/4000 with the diluent buffer was added at 100 µl/well. Then, the plate was incubated at room temperature for one hour. After washing the plate five times, a chromogenic substrate (Sigma) was added at 100 µl/well. The plate was then incubated at room temperature for 30 minutes. Absorbance at 405 nm (reference at 655 nm) was measured using the Model 3550 Microplate Reader (Bio-Rad Laboratories).

The modified antibodies (hA69-N97R, hA69-p18, and hA69-PF) used in Example 8 were assessed by the following procedure. After Factor IXa (Enzyme Research Laboratories) diluted to 1 µg/ml with a coating buffer (0.05 M carbonate-bicarbonate buffer, pH 9.6) was aliquoted (100 µl/well) into a Nunc-Immuno plate (a Nunc-Immuno^{™} 96 MicroWell^{™} MaxiSorp^{™} plate (Nalge Nunc Intemational)), the plate was incubated at 4°C overnight or for a longer period. After washing three times with PBS containing 0.05% Tween^{(R)} 20, a diluent buffer (Tris buffered saline containing Tween 20 (pH 8.0) (Sigma), 1% bovine serum albumin, 0.02% sodium azide) was added to the plate at 200 µl/well. Then, the plate was blocked at room temperature for two hours. After removal of the buffer, the purified antibodies diluted with the diluent buffer were added at 100 µl/well. The plate was then incubated overnight at 4°C. After washing the plate three times, alkaline phosphatase-labeled mouse anti-human IgG4 (Southern Biotechnology)) diluted to 1/500 with the diluent buffer was added at 100 µl/well. The plate was incubated at room temperature for two hours. After washing the plate five times, the BluePhos Microwell Phosphatase Substrates System (Kirkegaard & Perry Laboratories) was added as a substrate at 100 µl/well. The plate was then incubated at room temperature for about 30 minutes. Absorbance at 650 nm was measured using the Vmax Microplate Reader (Molecular Devices). As shown in Fig. 5, the results demonstrate that the antibodies in which the variable region had been modified to change the surface charge showed a binding activity comparable to that of the original antibodies before modification.

Furthermore, the functions of the humanized B26 antibody hB26-F123e4 and its modified form, hB26-p15, were assessed by assaying their binding activities to the antigen Factor X. Factor X (Enzyme Research Laboratories) diluted to 1 µg/ml with a coating buffer (100 mM sodium bicarbonate (pH 9.6), 0.02% sodium azide) was aliquoted (100 µl/well) into a Nunc-Immuno plate (a Nunc-Immuno^{™} 96 MicroWell^{™} MaxiSorp^{™} plate), and then the plate was incubated overnight at 4°C. After washing three times with PBS(-) containing Tween^{(R)} 20, the plate was blocked with a diluent buffer (50 mM Tris-HCl (pH 8.1), 1% bovine serum albumin, 1 mM MgCl₂, 0.15 M NaCl, 0.05% Tween^{(R)} 20, 0.02% sodium azide) at room temperature for two hours. After removal of the buffer, the purified antibodies diluted with the diluent buffer were added at 100 µl/well to the plate. The plate was incubated at room temperature for one hour. After the plate was washed three times, alkaline phosphatase-labeled goat anti-mouse IgG (BIOSOURCE) diluted to 1/4000 with the diluent buffer was added at 100 µl/well. The plate was then incubated at room temperature for one hour. After washing the plate five times, a chromogenic substrate (Sigma) was added at 100 µl/well. The plate was then incubated at room temperature for 30 minutes. Absorbance at 405 nm (reference at 655 nm) was measured using the Model 3550 Microplate Reader (Bio-Rad Laboratories). As shown in Fig. 6, the results demonstrated that the antibody in which the variable region had been modified to change the surface charge showed binding activity comparable to that of the original antibody before modification.

The findings described above demonstrate that the modifications of variable regions performed in the Examples have no influence on the antigen binding activity of the antibodies.

### [Example 8] Assessment of the prepared antibodies for pharmacokinetics 8-1. Test of pharmacokinetics using mice

ATF was obtained as a monoclonal antibody against human tissue factor, and is a humanized antibody comprising the constant regions of human IgG4. The origin of ATF is described in detail in WO99/051743. The amino acid sequences of its H chain and L chain variable regions are shown in SEQ ID NOs: 13 and 14, respectively. hA69-PF, BiAb, and hB26-PF prepared in Example 5, hA69-N97R, hA69-p18, hB26-e, and hB26-p15 prepared in Example 3, and ATF were assessed for the *in vivo* kinetics in mice (C57BL/6J; Charles River Japan, Inc.). ATF, hA69-PF, BiAb, and hB26-PF were intravenously administered once at 5 mg/kg to mice (C57BL/6J; Charles River Japan, Inc.). The blood was collected before administration and 15 minutes, two hours, eight hours, and one, two, four, seven, 11, 14, 21, and 28 days after administration. The collected blood was immediately centrifuged at 4°C and 15,000 rpm for 15 minutes to obtain plasma. The separated plasma was stored in a freezer at -20°C or below until use. Likewise, hA69-N97R, hA69-p18, hB26-F123e4, and hB26-p15 were intravenously administered once at 1 mg/kg to mice (C57BL/6J; Charles River Japan, Inc.). The blood was collected before administration and 15 minutes, two hours, eight hours, and one, two, five, seven, nine, 14, 21, and 28 days after administration. The collected blood was immediately centrifuged at 4°C and 15,000 rpm for 15 minutes to obtain plasma. The separated plasma was stored in a freezer at -20°C or below until use.

### 8-2. Measurement of plasma concentration by ELISA

Plasma concentrations in mice were determined by ELISA. Calibration curve samples of 6.4, 3.2, 1.6, 0.8, 0.4, 0.2, and 0.1 µg/ml plasma concentrations were prepared. The standard curve samples and mouse plasma samples to be tested were aliquoted into immunoplates (Nunc-Immuno MaxiSorp plates (Nalge nunc International) immobilized with an anti-human IgG (y-chain specific) F(ab')2 (Sigma). The samples were left to stand at room temperature for one hour, and then reacted with Goat Anti-Human IgG-BIOT (Southern Biotechnology Associates) and Streptavidin-alkaline phosphatase conjugate (Roche Diagnostics) in succession. The color development was carried out using BluePhos Microwell Phosphatase Substrates System (Kirkegaard & Perry Laboratories) as a substrate. The absorbance at 650 nm was measured using a microplate reader. The plasma concentrations in mice were calculated from the absorbance on the calibration curve using the analysis software SOFTmax PRO (Molecular Devices). The time courses of plasma concentrations of ATF, hA69-PF, BiAb, and hB26-PF are shown in Fig. 7.

### 8-3. Method for calculating pharmacokinetic data

The obtained data on the time courses of plasma concentrations were evaluated by a model-independent analysis using the pharmacokinetic analysis software WinNonlin (Pharsight) to calculate pharmacokinetic parameters (clearance (CL), half-life (T1/2)). T1/2 was calculated from plasma concentrations at the last three points or in the terminal phase automatically selected by WinNonlin. The determined pharmacokinetic parameters are shown in Table 2.

| Table 2 | | | | | |
|---|---|---|---|---|---|
| | | hA69-N97R | hA69-p18 | hA69-PF | ATF |
| pI | | 8.9 | 8.5 | 8.0 | 7.2 |
| CL | mL/h/kg | 0.412 | 0.300 | 0.204 | 0.136 |
| T1/2 | day | 12.6 | 15.0 | 18.7 | 26.1 |
| | | | | | |

| | | hB26-F123e4 | hB26-p15 | hB26-PF | BiAb |
|---|---|---|---|---|---|
| pI | | 8.7 | 9.0 | 9.2 | 8.7 |
| CL | mL/h/kg | 0.346 | 0.450 | 0.600 | 0.362 |
| T1/2 | day | 13.4 | 11.9 | 10.8 | 13.6 |

Furthermore, plots of antibody clearance (CL) and half-life (T1/2) relative to pI are shown in Fig. 8. While the respective antibodies used share the same constant region sequences, each of clearance (CL) and half-life (T1/2) is strongly correlated with pI. This shows that, as pI is lower, clearance is lower and half-life in blood is more prolonged. Thus, half-life in blood can be controlled by pI values even when antibodies share the same constant region sequences. Accordingly, it is suggested that the half-life in blood can be prolonged by decreasing pI or can be reduced by increasing pI. In this Example, it is demonstrated that the half-life in blood could be actually prolonged by decreasing pI through modification of surface amino acids (the modification sites are shown in Table 3) in the variable regions of hA69-N97R. Half-life in blood can be reduced by increasing pI through modification of surface amino acids (the modification sites are shown in Table 4) in the variable regions of hB26-F123e4. These findings suggest that pharmacokinetics of IgGs in blood can be controlled by changing charges of surface amino acids (for example, at positions H10, H12, H23, H39, H43, H97, and H105) in their variable regions through modifications.

**Table 3**

| Name | H1 | H12 | H23 | H27 | H43 | H97 | H103 | L99 |
|---|---|---|---|---|---|---|---|---|
| hA69-N97R | Pyr(Q) | K | K | G | Q | R | Q | G |
| hA69-p18 | Pyr(Q) | K | K | G | E | N | E | G |
| hA69-PF | E | V | T | Y | E | L | E | Q |
| | | * | * | | * | * | * | |

**Table 4**

| Name | H1 | H9 | H10 | H28 | H37 | H39 | H43 | H103 | L99 |
|---|---|---|---|---|---|---|---|---|---|
| hB26-F123e4 | Pyr(Q) | P | D | M | A | Q | Q | Q | G |
| hB26-p15 | Pyr(Q) | P | D | M | A | K | K | R | G |
| hB26-PF | E | A | Q | T | V | Q | K | R | Q |
| | | | * | | | * | * | * | |

In Tables 3 and 4 above, Pyr(Q) represents an N-terminal glutamine residue which is assumed to be pyro-glutamylated. Since the N-terminal amino group is protected, there is no significant charge difference between Pyr(Q) and E. Furthermore, sites of amino acid substitution which results in a pI shift are indicated by an asterisk.

The present invention discovered that the half-life in blood of an IgG could be prolonged or reduced by decreasing or increasing the pI of IgG through substitution of surface amino acids in the variable regions, respectively.

According to a non-patent document (Nat Biotechnol. 1997; 15: 637-640) on blood pharmacokinetics in mice, the half-life in blood (T1/2) could be prolonged by about 1.5 times by increasing the affinity for FcRn through modification of amino acids in the Fc in the constant region. Also in the present invention, by decreasing pI through modification of surface amino acids in variable regions, the half-life in blood (T1/2) could be prolonged by about 1.5 times in the comparison between hA69-N97R and hA69-PF sharing the same constant region sequences. Furthermore, when hA69-N97R is compared with hA69-PF and ATF, T1/2 of ATF with the lowest pI is longer by about 2.1 times than that of hA69-N97R. Thus, the half-life of hA69-N97R in blood can be further prolonged by decreasing its pI through additional modification of surface amino acids in the variable regions of hA69-N97R. When the antibodies used in this Example are compared to each other, the half-life in blood is different by about 2.4 times between hB26-PF with the highest pI and ATF with the lowest pI. Accordingly, the control of pharmacokinetics in blood through amino acid modifications in variable regions is expected to be more effective as compared to previous control techniques. Furthermore, the number of amino acid substitutions artificially introduced into constant regions is desired to be smaller from the viewpoint of antigenicity. Thus, the present invention, in which half-life in blood is controlled by modifying surface amino acids in variable regions, is expected to be useful in developing pharmaceuticals.

### Industrial Applicability

In a preferred embodiment of the methods of the present invention, amino acid substitutions are performed in variable regions, and thus the risk of antigenicity is low as compared to conventional methods that modify constant regions. Furthermore, the methods of the present invention can be more effective in prolonging the half-life in blood as compared to the conventional methods that modify constant regions. In addition, the half-life in blood of polypeptides comprising an FcRn-binding domain, such as IgG antibodies, can be controlled by controlling the surface charge in variable regions without changing structure or function (activity). Polypeptides comprising an FcRn-binding domain, which retain the original activity and whose half-life in blood is controlled, can be obtained by using the methods of the present invention.

## Claims

1. A method for producing a modified humanized IgG antibody comprising an FcRn-binding domain, having prolonged or reduced half-life in blood as compared to before said modification, wherein the method comprises:
(a) modifying a nucleic acid encoding a humanized IgG antibody to change the charge of at least one amino acid residue that can be exposed on the surface of the antibody, wherein said at least one amino acid residue is an amino acid residue in a heavy chain or light chain variable region of said antibody, wherein the change in charge of said at least one amino acid residue is achieved by amino acid substitution such that said modifying shifts the isoelectric point of said antibody;
(b) culturing a host cell to express the nucleic acid;
(c) collecting said modified antibody from the host cell culture;
(d) analyzing the isoelectric point of said modified humanized IgG antibody;
(e) assessing the binding activity of said modified humanized IgG antibody; and
(f) assessing whether said half-life has been prolonged or reduced by conducting kinetic tests;
wherein the modified humanized IgG antibody retains its binding activity.

2. The method of claim 1, wherein said at least one amino acid residue is selected from the group consisting of amino acid residues at positions 10, 12, 23, 39, 43, and 105 according to the Kabat's numbering system in the heavy chain of the IgG.

3. The method of claim 1 or 2, wherein said at least one amino acid residue is selected from amino acid residues of either group (a) or (b) below:
(a) glutamic acid (E) and aspartic acid (D); and
(b) lysine (K), arginine (R), and histidine (H).

## Patentansprüche

1. Verfahren zur Herstellung eines modifizierten humanisierten IgG-Antikörpers, umfassend eine FcRn-bindende Domäne, dessen Halbwertszeit im Blut im Vergleich zu vor der Modifizierung des Antikörpers verlängert oder reduziert ist, wobei das Verfahren umfasst:
(a) Modifizieren einer Nucleinsäure, die einen humanisierten IgG-Antikörper codiert, um die Ladung von mindestens einem Aminosäurerest zu ändern, der auf der Oberfläche des Antikörpers exponiert sein kann, wobei der mindestens eine Aminosäurerest ein Aminosäurerest in einer variablen Region einer schweren Kette oder einer leichten Kette des Antikörpers ist und wobei die Änderung der Ladung des mindestens einen Aminosäurerests durch Aminosäuresubstitution erreicht wird, so dass die Modifizierung den isoelektrischen Punkt des Antikörpers verschiebt;
(b) Züchten einer Wirtszelle, um die Nucleinsäure zu exprimieren;
(c) Gewinnen des modifizierten Antikörpers aus der Wirtszellkultur;
(d) Analysieren des isoelektrischen Punkts des modifizierten humanisierten IgG-Antikörpers;
(e) Beurteilen der Bindungsaktivität des modifizierten humanisierten IgG-Antikörpers; und
(f) Beurteilen durch Durchführung kinetischer Tests, ob die Halbwertszeit verlängert oder verkürzt ist;
wobei der modifizierte humanisierte IgG-Antikörper seine Bindungsaktivität beibehält.

2. Verfahren nach Anspruch 1, wobei der mindestens eine Aminosäurerest ausgewählt ist aus der Gruppe bestehend aus den Aminosäureresten an den Positionen 10, 12, 23, 39, 43 und 105 gemäß dem Kabat-Nummerierungssystem in der schweren Kette des IgG.

3. Verfahren nach Anspruch 1 oder 2, wobei der mindestens eine Aminosäurerest aus den Aminosäureresten einer der Gruppen (a) oder (b) unten ausgewählt ist:
(a) Glutaminsäure (E) und Asparaginsäure (D); und
(b) Lysin (K), Arginin (R) und Histidin (H).

## Revendications

1. Méthode pour produire un anticorps IgG humanisé modifié comprenant un domaine de liaison à FcRn, ayant une demi-vie prolongée ou réduite dans le sang en comparaison au cas avant ladite modification, laquelle méthode comprend :
(a) la modification d'un acide nucléique codant un anticorps IgG humanisé pour changer la charge d'au moins un résidu d'acide aminé qui peut être exposé sur la surface de l'anticorps, dans laquelle ledit au moins un résidu d'acide aminé est un résidu d'acide aminé dans une région variable de chaîne lourde ou de chaîne légère dudit anticorps, dans laquelle le changement de charge dudit au moins un résidu d'acide aminé est obtenu par substitution d'acide aminé de façon que ladite modification décale le point isoélectrique dudit anticorps ;
(b) la culture d'une cellule hôte pour qu'elle exprime l'acide nucléique ;
(c) la collecte dudit anticorps modifié à partir de la culture de la cellule hôte ;
(d) l'analyse du point isoélectrique dudit anticorps IgG humanisé modifié ;
(e) la détermination de l'activité de liaison dudit anticorps IgG humanisé modifié ; et
(f) la détermination que ladite demi-vie a été prolongée ou réduite par mise en œuvre de tests cinétiques ;
dans laquelle l'anticorps IgG humanisé modifié conserve son activité de liaison.

2. Méthode selon la revendication 1, dans laquelle ledit au moins un résidu d'acide aminé est choisi dans le groupe constitué par les résidus d'acides aminés aux positions 10, 12, 23, 39, 43, et 105 conformément au système de numérotation de Kabat dans la chaîne lourde de l'IgG.

3. Méthode selon la revendication 1 ou 2, dans laquelle ledit au moins un résidu d'acide aminé est choisi parmi les résidus d'acides aminés de l'un ou l'autre des groupes (a) et (b) ci-dessous :
(a) l'acide glutamique (E) et l'acide aspartique (D) ; et
(b) la lysine (K), l'arginine (R) et l'histidine (H).
